# EUROPEAN PATENT SPECIFICATION

(11) **EP 1 503 737 B1**
(45) Date of publication and mention of the grant of the patent: **07.01.2009**
(21) Application number: 03728678.8
(22) Date of filing: 05.05.2003
(51) Int. Cl.: A61K 9/14, A61K 31/70, A61K 47/24, A61K 47/18, A61K 47/32, A61K 47/34, A61K 47/42

(54) **NANOPARTICULATE NYSTATIN FORMULATIONS**
NYSTATIN-NANOPARTIKELZUSAMMENSETZUNGEN
FORMULATIONS DE NYSTATINE NANOPARTICULAIRES

(30) Priority: 06.05.2002 US 377599 P; 07.05.2002 US 380008 P; 04.11.2002 US 423385 P
(43) Date of publication of application: 09.02.2005
(73) Proprietor: Elan Pharma International Limited, Athlone County Westmeath (IE)
(72) Inventor: CUNNINGHAM, James, Malvern, PA 19355 (US); MERISKO-LIVERSIDGE, Elaine, West Chester, PA 19380 (US)
(74) Representative: Wichmann, Hendrik
(86) International application number: PCT/US2003/013833
(87) International publication number: WO 2003/094894

(56) References cited:
- EP-A- 0 499 299
- WO-A-00/47187
- WO-A-00/53164
- WO-A-01/26635
- WO-A-01/68102
- US-A- 5 510 118

## Description

### FIELD OF THE INVENTION

The present invention relates to a nanoparticulate composition comprising nystatin and at least one surface stabilizer associated with or adsorbed to the surface of the drug, as defined in the claims. The nanoparticulate nystatin particles have an effective average particle size of less than about 2000 nm.

### BACKGROUND OF THE INVENTION

### A. Background Regarding "Nanoparticulate Compositions.

Nanoparticulate compositions, first described in U.S. Patent No. 5,145,684 ("the '684 patent"), are particles consisting of a poorly soluble therapeutic or diagnostic agent having associated with or adsorbed to the surface thereof a non-crosslinked surface stabilizer. The '684 patent does not describe nanoparticulate compositions of nystatin.

Methods of making nanoparticulate compositions are described, for example, in U.S. Patent Nos. 5,518,187 and 5,862,999, both for "Method of Grinding Pharmaceutical Substances;" U.S. Patent No. 5,718,388, for "Continuous Method of Grinding Pharmaceutical Substances;" and U.S. Patent No. 5,510,118 for "Process of Preparing Therapeutic Compositions Containing Nanoparticles."

Nanoparticulate compositions are also described, for example, in U.S. Patent Nos. 5,298,262 for "Use of Ionic Cloud Point Modifiers to Prevent Particle Aggregation During Sterilization," 5,302,401 for "Method to Reduce Particle Size Growth During Lyophilization;" 5,318,767 for "X-Ray Contrast Compositions Useful in Medical Imaging;" 5,326,552 for "Novel Formulation For Nanoparticulate X-Ray Blood Pool Contrast Agents Using High Molecular Weight Non-ionic Surfactants;" 5,328,404 for "Method of X-Ray Imaging Using Iodinated Aromatic Propanedioates;" 5,336,507 for "Use of Charged Phospholipids to Reduce Nanoparticle Aggregation;" 5,340,564 for "Formulations Comprising Olin 10-G to Prevent Particle Aggregation and Increase Stability;" 5,346,702 for "Use of Non-Ionic Cloud Point Modifiers to Minimize Nanoparticulate Aggregation During Sterilization;" 5,349,957 for "Preparation and Magnetic Properties of Very Small Magnetic-Dextran Particles;" 5,352,459 for "Use of Purified Surface Modifiers to Prevent Particle Aggregation During Sterilization;" 5,399,363 and 5,494,683, both for "Surface Modified Anticancer Nanoparticles;" 5,401,492 for "Water Insoluble Non-Magnetic Manganese Particles as Magnetic Resonance Enhancement Agents;" 5,429,824 for "Use of Tyloxapol as a Nanoparticulate Stabilizer," 5,447,710 for "Method for Making Nanoparticulate X-Ray Blood Pool Contrast Agents Using High Molecular Weight Non-ionic Surfactants;" 5,451,393 for "X-Ray Contrast Compositions Useful in Medical Imaging;" 5,466,440 for "Formulations of Oral Gastrointestinal Diagnostic X-Ray Contrast Agents in Combination with Pharmaceutically Acceptable Clays;" 5,470,583 for "Method of Preparing Nanoparticle Compositions Containing Charged Phospholipids to Reduce Aggregation;" 5,472,683 for "Nanoparticulate Diagnostic Mixed Carbamic Anhydrides as X-Ray Contrast Agents for Blood Pool and Lymphatic System Imaging;" 5,500,204 for "Nanoparticulate Diagnostic Dimers as X-Ray Contrast Agents for Blood Pool and Lymphatic System Imaging;" 5,518,738 for "Nanoparticulate NSAID Formulations;" 5,521,218 for "Nanoparticulate Iododipamide Derivatives for Use as X-Ray Contrast Agents;" 5,525,328 for "Nanoparticulate Diagnostic Diatrizoxy Ester X-Ray Contrast Agents for Blood Pool and Lymphatic System Imaging;" 5,543,133 for "Process of Preparing X-Ray Contrast Compositions Containing Nanoparticles;" 5,552,160 for "Surface Modified UNSAID Nanoparticles;" 5,560,931 for "Formulations of Compounds as Nanoparticulate Dispersions in Digestible Oils or Fatty Acids;" 5,565,188 for "Polyalkylene Block Copolymers as Surface Modifiers for Nanoparticles;" 5,569,448 for "Sulfated Non-ionic Block Copolymer Surfactant as Stabilizer Coatings for Nanoparticle Compositions;" 5,571,536 for "Formulations of Compounds as Nanoparticulate Dispersions in Digestible Oils or Fatty Acids;" 5,573,749 for "Nanoparticulate Diagnostic Mixed Carboxylic Anydrides as X-Ray Contrast Agents for Blood Pool and Lymphatic System Imaging;" 5,573,750 for "Diagnostic Imaging X-Ray Contrast Agents;" 5,573,783 for "Redispersible Nanoparticulate Film Matrices With Protective Overcoats;" 5,580,579 for "Site-specific Adhesion Within the GI Tract Using Nanoparticles Stabilized by High Molecular Weight, Linear Poly(ethylene Oxide) Polymers;" 5,585,108 for "Formulations of Oral Gastrointestinal Therapeutic Agents in Combination with Pharmaceutically Acceptable Clays;" 5,587,143 for "Butylene Oxide-Ethylene Oxide Block Copolymers Surfactants as Stabilizer Coatings for Nanoparticulate Compositions;" 5,591,456 for "Milled Naproxen with Hydroxypropyl Cellulose as Dispersion Stabilizer," 5,593,657 for "Novel Barium Salt Formulations Stabilized by Non-ionic and Anionic Stabilizers;" 5,622,938 for "Sugar Based Surfactant for Nanocrystals;" 5,628,981 for "Improved Formulations of Oral Gastrointestinal Diagnostic X-Ray Contrast-Agents and Oral Gastrointestinal Therapeutic Agents;" 5,643,552 for "Nanoparticulate Diagnostic Mixed Carbonic Anhydrides as X-Ray Contrast Agents for Blood Pool and Lymphatic System Imaging;" 5,718,388 for "Continuous Method of Grinding Pharmaceutical Substances;" 5,718,919 for "Nanoparticles Containing the R(-)Enantiomer of Ibuprofen;" 5,747,001 for "Aerosols Containing Beclomethasone Nanoparticle Dispersions;" 5,834,025 for "Reduction of Intravenously Administered Nanoparticulate Formulation Induced Adverse Physiological Reactions;" 6,045,829 "Nanocrystalline Formulations of Human Immunodeficiency Virus (HIV) Protease Inhibitors Using Cellulosic Surface Stabilizers;" 6,068,858 for "Methods of Making Nanocrystalline Formulations of Human Immunodeficiency Virus (HIV) Protease Inhibitors Using Cellulosic Surface Stabilizers," 6,153,225 for "Injectable Formulations of Nanoparticulate Naproxen;" 6,165,506 for "New Solid Dose Form of Nanoparticulate Naproxen;" 6,221,400 for "Methods of Treating Mammals Using Nanocrystalline Formulations of Human Immunodeficiency Virus (HIV) Protease Inhibitors;" 6,264,922 for "Nebulized Aerosols Containing Nanoparticle Dispersions;" 6,267,989 for "Methods for Preventing Crystal Growth and Particle Aggregation in Nanoparticle Compositions;" 6,270,806 for "Use of PEG-Derivatized Lipids as Surface Stabilizers for Nanoparticulate Compositions;" and 6,316,029 for "Rapidly Disintegrating Solid Oral Dosage Form." In addition, U.S. Patent Application No. 20020012675 A1, published on January 31, 2002, for "Controlled Release Nanoparticulate Compositions," describes nanoparticulate compositions.

Amorphous small particle compositions are described, for example, in U.S. Patent Nos. 4,783,484 for "Particulate Composition and Use Thereof as Antimicrobial Agents;" 4,826,689 for "Method for Making Uniformly Sized Particles from Water-Insoluble Organic Compounds;" 4,997,454 for "Method for Making Uniformly-Sized Particles From Insoluble Compounds;" 5,741,522 for "Ultrasmall, Non-aggregated Porous Particles of Uniform Size for Entrapping Gas Bubbles Within and Methods;" and 5,776,496, for "Ultrasmall Porous Particles for Enhancing Ultrasound Back Scatter."

### B. Background Regarding Nystatin

Nystatin is a poorly water-soluble antimycotic polyene antibiotic obtained from *Streptomyces noursei.* Nystatin is poorly absorbed. Nystatin A is a macrocyclic lactone containing a ketal ring, an all-*trans* polyene system, and a mycosamine (3-amino-3-deoxy-rhamnose) moiety. Nystatin A has a molecular formula of C₄₇H₇₅NO₁₇ and a molecular weight of 926.11. *See* The Physicians' Desk Reference, 56th Edition, pp. 2445 and 2608 (2002).

Nystatin is both fungistatic and fungicidal *in vitro* against a wide variety of yeasts and yeast-like fungi, including *Candida albicans, C. parapsilosis, C. tropicalis, C. guilliermondi, C. pseudotropicalis, C. krusei, Torulopsis glabrata, Tricophyton rubrum,* and *T. mentagrophytes.* Nystatin is fungistatic *in vitro* against a variety of fungi including the principal fungi pathogenic in man. Orally, it inhibits the overgrowth of candida albicans and other yeast-like flora in the mouth and intestinal tract.

Nystatin exerts its therapeutic effect by binding to sterols in the cell membrane of sensitive fungi with a resultant change in membrane permeability allowing leakage of intracellular components. Nystatin exhibits no appreciable activity against bacteria, protozoa, trichomonads, or viruses.

Nystatin is not absorbed from intact skin or mucous membrane. When administered orally nystatin is virtually not absorbed by the digestive tract. Therefore, when therapeutic and prophylactic doses are given as recommended, no detectable blood levels are obtained. Trace blood levels may be detected with the administration of very high doses of nystatin. The non absorbed nystatin is passed unchanged in the faeces.

The drug is useful in treating infections of susceptible organisms. Oral candidiasis, in particular, is a common affliction of immunocompromised patients. Nystatin is indicated in the therapy of all infections caused by susceptible microorganisms in those patients in whom candidal (monilial) infections are most likely to complicate therapy. It is useful in the treatment of patients receiving high and prolonged dosages of antibiotics, with or without corticosteroid therapy, elderly or debilitated patients, diabetics, patients receiving radiation therapy and antineoplastic drugs and patients with prior or recurrent candidal infections. It is also indicated in the treatment of women with infections who are taking oral contraceptives or have histories of recurrent vaginal moniliasis. It is indicated in the treatment of respiratory infections, such as pneumonia, bronchitis, tonsillitis, pharyngitis, laryngitis, and otitis media; genitourinary infections such as cystitis, pyelonephritis, urethritis, and gonorrhoea; and skin and soft tissue infections such as pyoderma, erysipelas, lymphangitis, and cellulitis.

Marketed forms of Nystatin include Nystatin Vaginal Tablets, 100,000 units, by Odyssey Pharmaceuticals, Inc. (East Hanover, NJ). The tablets are effective for the local treatment of vulvovaginal candidiasis (moniliasis). The usual dosage is one tablet (100,000 units nystatin) daily for two weeks, and each tablet is deposited high in the vagina by means of an applicator. The Physicians' Desk Reference, 56th Edition, at 2445.

Other marketed forms of nystatin include PADDOCK^{™} Nystatin, which is an oral suspension, and NYSTOP®, which is a nystatin topical powder, both manufactured by Paddock Laboratories, Inc. (Minneapolis, MN). The oral suspension is a ready-to-use, non-sterile powder for oral administration which contains no excipients or preservatives. It is available in containers of 50 million, 150 million, 500 million, 2 billion, and 5 billion units. Each milligram of nystatin contains a minimum of 5,000 units. *The Physicians' Desk Reference,* 56^{th} Edition, at 2608. The nystatin topical powder, marketed under the tradename NYSTOP®, is for dermatologic use. The powder contains 100,000 USP nystatin units per gram dispersed in talc, and the product is marketed in 15 g plastic squeeze bottles. It is indicated for the treatment of cutaneous or mucocutaneous mycotic infections caused by *Candida albicans* and other suseptible *Candida* species. *Id.*

In addition, nystatin is marketed by Geneva Pharmaceuticals, Inc. (Broomfield, CO) in an oral suspension. Other marketed products containing nystatin include CANSTAT® suspension (Wyeth South Africa Ltd.; for the specific treatment and prevention of *Candida albicans* moniliasis of the oral cavity), CANSTAT® topical cream (Wyeth South Africa Ltd.; for the treatment of cutaneous mycotic infections caused by Candida species (Monilia)), and CANSTAT® vaginal tablets (Wyeth South Africa Ltd.; for the specific treatment for *Candida albicans* moniliasis of the vaginal cavity and as an adjuvant preventive against secondary infections during antibiotic or corticosteroid therapy).

The current marketed forms of nystatin have several drawbacks. For example, aqueous suspensions suffer from a number of compliance-related drawbacks. For maximum efficacy, the suspension must be dosed multiple times daily, must be retained in the mouth for prolonged periods, and has an unpleasant taste which in some cases is covered by addition of sucrose, a promoter of tooth decay.

There is a need in the art for nystatin formulations which can decrease frequency of dosing and improve clinical efficacy. The present invention satisfied these needs.

### SUMMARY OF THE INVENTION

The present invention relates to nanoparticulate compositions comprising nystatin. The compositions comprise nystatin and at least one surface stabilizer associated with or adsorbed to the surface of the nystatin particles, as defined in the claims. The nanoparticulate nystatin particles have an effective average particle size of less than about 2000 nm.

Another aspect of the invention is directed to pharmaceutical compositions comprising a nanoparticulate nystatin composition of the invention. The pharmaceutical compositions preferably comprise nystatin, at least one surface stabilizer, and a pharmaceutically acceptable carrier, as well as any desired excipients, as defined in the claims.

The present invention is also directed towards nystatin compositions with improved characteristics as compared to traditional forms of nystatin. Such characteristics or benefits of the compositions of the invention as compared to conventional prior art micronized or solubilized forms of nystatin may include, but are not limited to: (1) faster onset of action; (2) a potential decrease in the frequency of dosing; (3) smaller doses of nystatin required to obtain the same pharmacological effect as compared to conventional microcrystalline forms of nystatin; (4) improved performance characteristics for oral, intravenous, subcutaneous, or intramuscular injection, such as higher dose loading and smaller tablet or liquid dose volumes; (5) low viscosity liquid nanoparticulate nystatin dosage forms; (6) for liquid nanoparticulate nystatin compositions having a low viscosity - better subject compliance due to the perception of a lighter formulation which is easier to consume and digest; (7) for liquid nanoparticulate nystatin compositions having a low viscosity - ease of dispensing because one can use a cup or a syringe; (8) the nanoparticulate nystatin particles present in the compositions of the invention readily redisperse following administration; (9) the nystatin compositions of the invention can be formulated in a dried form which readily redisperses; (10) the nanoparticulate nystatin compositions preferably exhibit an increased rate of dissolution as compared to conventional microcrystalline forms of nystatin; (11) a bioadhesive nystatin formulation can coat the gut, the oral cavity, or the desired site of application, and be retained for a period of time, thereby eliminating the need for the patient to hold the whole dose in their mouth and increasing the efficacy of the drug as well as eliminating or decreasing the frequency of dosing; (12) the nanoparticulate nystatin compositions can be used in conjunction with other active agents; (13) the nanoparticulate nystatin compositions can be sterile filtered; (14) the nanoparticulate nystatin compositions are suitable for parenteral administration; (15) the nystatin compositions may be formulated as a ready to use colloidal dispersion rather than as a suspension that may settle or must be prepared immediately before dosing; (16) the bad taste associated with current conventional nystatin compositions may be reduced or eliminated; and (17) the nanoparticulate nystatin compositions do not require organic solvents or pH extremes.

This invention further discloses a method of making a nanoparticulate nystatin composition according to the invention. Such a method comprises contacting nystatin and at least one surface stabilizer for a time and under conditions sufficient to provide a nanoparticulate nystatin composition as defined in the claims. The one or more surface stabilizers can be contacted with nystatin either before, during, or after size reduction of the nystatin.

The present invention is also directed to methods of treatment using the nanoparticulate compositions of the invention for fungal infections of organisms including, but not limited to, *Candida albicans, C. parapsilosis, C. tropicalis, C. guilliermondi, C. pseudotropicalis, C. krusei, Torulopsis glabrala, Tricophyton rubrum,* and *T. mentagrophytes.* The fungal infections can be topical or non-topical, and include, but are not limited to, vaginal infections, skin infections, and mucous membrane infections, including oral infections.

Both the foregoing general description and the following detailed description are exemplary and explanatory and are intended to provide further explanation of the invention as claimed. Other objects, advantages, and novel features will be readily apparent to those skilled in the art from the following detailed description of the invention.

### BRIEF DESCRIPTION OF THE FIGURES

- FIGURE 1:: Shows representative photomicrographs of nystatin crystals before (Fig. 1A) and after (Fig. 1B) milling;
- FIGURE 2:: Shows the results of monitoring the particle size stability over time at 5°C (solid line), 25°C (dashed line), and 40°C (dotted line) for four nanoparticulate nystatin formulations. Formulation 1 (Figure 2A) comprised sodium deoxycholate as a surface stabilizer; Formulation 2 (Figure 2B) comprised DSPE-PEG(2000)-Amine as a surface stabilizer; Formulation 3 (Figure 2C) comprised Poly(Lys, Trp) 4:1 Hydrobromide as a surface stabilizer; and Formulation 4 (Figure 2D) comprised povidone-iodine as a surface stabilizer; and
- FIGURE 3:: Shows representative micrographs of cells with anionic particles (Fig. 3A) and cationic particles (Fig. 3B).

### DETAILED DESCRIPTION OF THE INVENTION

The present invention is directed to nanoparticulate compositions comprising nystatin as defined in the claims. The compositions comprise nystatin and at least one surface stabilizer associated with or adsorbed to the surface of the drug. The nanoparticulate nystatin particles have an effective average particle size of less than about 2000 nm.

As taught in the '684 patent, not every combination of surface stabilizer and active agent will result in a stable nanoparticulate composition. It was surprisingly discovered that stable nystatin nanoparticulate formulations can be made.

The current formulations of nystatin for oral candidiasis suffer from the following problems: (1) the poor solubility of the drug necessitates making a suspension in water for administration; (2) since the medication is only effective when in the mouth the dose must be held in the mouth and swished or gargled for as long as the patient is reasonably able; (3) dosing must be repeated up to 5 times each day, resulting in patient compliance issues; (4) the oral suspension is unpalatable, with a thick and viscous consistency and a very bad taste, which may again limit patient compliance; and (5) the current marketed nystatin liquid dosage formulation must be retained in the mouth for a prolonged period to obtain the best therapeutic effect, which again results in patient compliance issues. Factor (4) has been addressed in some current formulations by the addition of sucrose and other sweeteners, but sucrose in particular is not ideal from a dental standpoint.

The present invention overcomes problems encountered with the prior art nystatin formulations. Specifically, the nanoparticulate nystatin formulations of the invention may offer the following advantages: (1) faster onset of action; (2) a potential decrease in the frequency of dosing; (3) smaller doses of nystatin required to obtain the same pharmacological effect as compared to conventional microcrystalline forms of nystatin; (4) improved performance characteristics for oral, intravenous, subcutaneous, or intramuscular injection, such as higher dose loading and smaller tablet or liquid dose volumes; (5) low viscosity liquid nanoparticulate nystatin dosage forms; (6) for liquid nanoparticulate nystatin compositions having a low viscosity - better subject compliance due to the perception of a lighter formulation which is easier to consume and digest; (7) for liquid nanoparticulate nystatin compositions having a low viscosity- ease of dispensing because one can use a cup or a syringe; (8) the nanoparticulate nystatin particles present in the compositions of the invention readily redisperse following administration; (9) the nystatin compositions of the invention can be formulated in a dried form which readily redisperses; (10) the nanoparticulate nystatin compositions preferably exhibit an increased rate of dissolution as compared to conventional microcrystalline forms of nystatin; (11) a bioadhesive nystatin formulation can coat the gut, the oral cavity, or the desired site of application, and be retained for a period of time, thereby eliminating the need for the patient to hold the whole dose in their mouth and increasing the efficacy of the drug as well as eliminating or decreasing the frequency of dosing; (12) the nanoparticulate nystatin compositions can be used in conjunction with other active agents; (13) the nanoparticulate nystatin compositions can be sterile filtered; (14) the nanoparticulate nystatin compositions are suitable for parenteral administration; (15) the nystatin compositions may be formulated as a ready to use colloidal dispersion rather than as a suspension that may settle or must be prepared immediately before dosing; (16) the bad taste associated with current conventional nystatin compositions may be reduced or eliminated; and (17) the nanoparticulate nystatin compositions do not require organic solvents or pH extremes.

The present invention is described herein using several definitions, as set forth below and throughout the application.

As used herein, "about" will be understood by persons of ordinary skill in the art and will vary to some extent on the context in which it is used. If there are uses of the term which are not clear to persons of ordinary skill in the art given the context in which it is used, "about" will mean up to plus or minus 10% of the particular term.

As used herein with reference to stable drug particles, 'stable' means that drug particles do not appreciably flocculate or agglomerate due to interparticle attractive forces or otherwise increase in particle size.

"Conventional active agents or drugs" refers to non-nanoparticulate compositions of active agents or solubilized active agents or drugs. Non-nanoparticulate active agents have an effective average particle size of greater than about 2 microns.

'Therapeutically effective amount' as used herein with respect to a drug dosage, shall mean that dosage that provides the specific pharmacological response for which the drug is administered in a significant number of subjects in need of such treatment. It is emphasized that 'therapeutically effective amount,' administered to a particular subject in a particular instance will not always be effective in treating the diseases described herein, even though such dosage is deemed a 'therapeutically effective amount' by those skilled in the art. It is to be further understood that drug dosages are, in particular instances, measured as oral dosages, or with reference to drug levels as measured in blood.

### A. Preferred Characteristics of the Nanoparticulate Nystatin Compositions of the Invention

### 1. Fast Onset of Activity

The use of conventional formulations of nystatin is not ideal due to delayed onset of action. In contrast, the nanoparticulate nystatin compositions of the invention exhibit faster therapeutic effects.

### 2, Frequency of Dosing and Dosage Quantity

The nystatin compositions of the invention may be administered less frequently and at lower doses in dosage forms such as liquid dispersions, powders, sprays, solid re-dispersable dosage forms, ointments, creams, *etc*. Exemplary types of formulations useful in the present invention include, but are not limited to, liquid dispersions, gels, aerosols (pulmonary and nasal), ointments, creams, solid dose forms, *etc.* of nanoparticulate nystatin. Lower dosages can be used because the small particle size of the nystatin particles ensure greater absorption, and in the case of bioadhesive nanoparticulate nystatin compositions, the nystatin is retained at the desired site of application for a longer period of time as compared to conventional nystatin dosage forms.

The compositions of the invention exhibit improved performance characteristics for oral, intravenous, subcutaneous, or intramuscular injection, such as higher dose loading and smaller tablet or liquid dose volumes.

In one embodiment of the invention, the therapeutically effective amount of the nanoparticulate nystatin compositions is 1/6, 1/5, ¼, 1/3^{rd}, or ½ of the therapeutically effective amount of a standard commercial nystatin formulation.

Conventional nystatin formulations used in treating infections of the oral cavity caused by *Candida (monilia) albicans* are generally dosed for infants at 1 -2 mL (20 -40 drops) (100,000 -200,000 units) four times daily taken orally and to be swallowed. In newborns the usual prophylactic dosage is 1 mL (20 drops) (100,000 units) daily to be given directly on the tongue. For children and adults the usual therapeutic dose is 1 -2 mL (20 to 40 drops) (100,000 to 200,000 units) four times daily. This dose may be increased to 4 to 6 mL (80 -120 drops) (400,000 to 600,000 units) four times daily. The oral suspension should not be swallowed directly but rather be swirled around the oral cavity for a brief period of time prior to swallowing.

In contrast, the liquid dispersion compositions of the invention can be administered less frequently and at lower doses in dosage forms such as liquid dispersions, powders, solid re-dispersable dosage forms, etc.

### 3. Low Viscosity

Typical liquid commercial formulations of nystatin are relatively large volume, highly viscous substances that are not well accepted by patient populations. Moreover, viscous solutions can be problematic in parenteral administration because these solutions require a slow syringe push and can stick to tubing. In addition, conventional formulations of poorly water-soluble active agents, such as nystatin, tend to be unsafe for intravenous administration techniques, which are used primarily in conjunction with highly water-soluble substances.

Liquid dosage forms of the nanoparticulate nystatin compositions of the invention provide significant advantages over conventional liquid nystatin dosage forms. The low viscosity and silky texture of liquid dosage forms of the nanoparticulate nystatin compositions of the invention results in advantages in both preparation and use. These advantages include, for example: (1) better subject compliance due to the perception of a lighter formulation which is easier to consume and digest; (2) ease of dispensing because one can use a cup or a syringe; (3) potential for formulating a higher concentration of nystatin resulting in a smaller dosage volume and thus less volume for the subject to consume; and (4) easier overall formulation concerns.

Liquid nystatin dosage forms which are easier to consume are especially important when considering juvenile patients, terminally ill patients, and elderly patients. Viscous or gritty formulations, and those that require a relatively large dosage volume, are not well tolerated by these patient populations.

The viscosities of liquid dosage forms of nanoparticulate nystatin according to the invention are preferably less than about 1/200, less than about 1/175, less than about 1/150, less than about 1/125, less than about 1/100, less than about 1/75, less than about 1/50, or less than about 1/25 of existing commercial liquid oral nystatin compositions at about the same concentration per ml of nystatin.

Typically the viscosity of liquid nanoparticulate nystatin dosage forms of the invention, at a shear rate of 0.1 (1/s), is from about 2000 mPa s to about 1 mPa s, from about 1900 mPa·s to about 1 mPa·s, from about 1800 mPa·s to about 1 mPa·s, from about 1700 mPa·s to about 1 mPa·s, from about 1600 mPa·s to about 1 mPa·s, from about 1500 mPa·s to about 1 mPa·s, from about 1400 mPa·s to about 1 mPa·s, from about 1300 mPa·s to about 1 mPa·s, from about 1200 mPa·s to about 1 mPa·s, from about 1100 mPa·s to about 1 mPa·s, from about 1000 mPa·s to about 1 mPa·s, from about 900 mPa·s to about 1 mPa·s, from about 800 mPa·s to about 1 mPa·s, from about 700 mPa·s to about 1 mPa·s, from about 600 mPa·s to about 1 mPa·s, from about 500 mPa·s to about 1 mPa·s, from about 400 mPa·s to about I mPa·s, from about 300 mPa·s to about 1 mPa·s, from about 200 mPa·s to about 1 mPa·s, from about 175 mPa·s to about 1 mPa·s, from about 150 mPa·s to about 1 mPa·s, from about 125 mPa·s to about 1 mPa·s, from about 100 mPa·s to about 1 mPa·s, from about 75 mPa·s to about 1 mPa·s, from about 50 mPa·s to about 1 mPa·s, from about 25 mPa·s to about 1 mPa·s, from about 15 mPa·s to about 1 mPa·s, from about 10 mPa·s to about 1 mPa·s, and from about 5 mPa·s to about 1 mPa·s. Such a viscosity is much more attractive for subject consumption and may lead to better overall subject compliance.

Viscosity is concentration and temperature dependent. Typically, a higher concentration results in a higher viscosity, while a higher temperature results in a lower viscosity. Viscosity as defined above refers to measurements taken at about 20°C. (The viscosity of water at 20°C is 1 mPa s.) The invention encompasses equivalent viscosities measured at different temperatures.

Another important aspect of the invention is that the nanoparticulate nystatin compositions of the invention are not turbid. "Turbid," as used herein refers to the property of particulate matter that can be seen with the naked eye or that which can be felt as "gritty." The nanoparticulate nystatin compositions of the invention can be poured out of or extracted from a container as easily as water, whereas a conventional standard commercial (*i.e.*, non-nanoparticulate or solubilized) nystatin liquid dosage form exhibits notably more "sluggish" characteristics.

The liquid formulations of this invention can be formulated for dosages in any volume but preferably equivalent or smaller volumes than existing commercial formulations.

### 4. Redispersibility Profiles of the Nanoparticulate Nystatin Compositions of the Invention

An additional feature of the nanoparticulate nystatin compositions of the invention is that the compositions redisperse such that the effective average particle size of the redispersed nystatin particles is less than about 2 microns. The redispersibility is observed with both solid dose and liquid dose formulations of nanoparticulate nystatin. This is significant, as if upon administration the nanoparticulate nystatin particles present in the compositions of the invention did not redisperse to a substantially nanoparticulate particle size, then the dosage form may lose the benefits afforded by formulating nystatin into a nanoparticulate particle size.

This is because nanoparticulate nystatin compositions benefit from the small particle size of nystatin; if the nanoparticulate nystatin particles do not redisperse into the small particle sizes upon administration, then "clumps" or agglomerated nystatin particles are formed. With the formation of such agglomerated particles, the bioavailability of the dosage form may fall.

The nanoparticulate nystatin compositions also preferably exhibit an increased rate of dissolution as compared to conventional microcrystalline forms of nystatin.

Preferably, the redispersed nystatin particles of the invention have an effective average particle size, by weight, of less than about 2 microns, less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, or less than about 50 nm, as measured by light-scattering methods, microscopy, or other appropriate methods.

Moreover, the nanoparticulate nystatin compositions of the invention exhibit dramatic redispersion of the nanoparticulate nystatin particles upon administration to a mammal, such as a human or animal, as demonstrated by reconstitution in a biorelevant aqueous media. Such biorelevant aqueous media can be any aqueous media that exhibit the desired ionic strength and pH, which form the basis for the biorelevance of the media. The desired pH and ionic strength are those that are representative of physiological conditions found in the human body. Such biorelevant aqueous media can be, for example, aqueous electrolyte solutions or aqueous solutions of any salt, acid, or base, or a combination thereof, which exhibit the desired pH and ionic strength.

Biorelevant pH is well known in the art. For example, in the stomach, the pH ranges from slightly less than 2 (but typically greater than 1) up to 4 or 5. In the small intestine the pH can range from 4 to 6, and in the colon it can range from 6 to 8. Biorelevant ionic strength is also well known in the art. Fasted state gastric fluid has an ionic strength of about 0.1M while fasted state intestinal fluid has an ionic strength of about 0.14. *See e.g.,* Lindahl et al., "Characterization of Fluids from the Stomach and Proximal Jejunum in Men and Women," Pharm. Res., 14 (4): 497-502 (1997).

It is believed that the pH and ionic strength of the test solution is more critical than the specific chemical content. Accordingly, appropriate pH and ionic strength values can be obtained through numerous combinations of strong acids, strong bases, salts, single or multiple conjugate acid-base pairs (*i.e.*, weak acids and corresponding salts of that acid), monoprotic and polyprotic electrolytes, *etc*.

Representative electrolyte solutions can be, but are not limited to, HCl solutions, ranging in concentration from about 0.001 to about 0.1 M, and NaCl solutions, ranging in concentration from about 0.001 to about 0.1 M, and mixtures thereof. For example, electrolyte solutions can be, but are not limited to, about 0.1 M HCl or less, about 0.01 M HCl or less, about 0.001 M HCl or less, about 0.1 M NaCl or less, about 0.01 M NaCl or less, about 0.001 M NaCl or less, and mixtures thereof. Of these electrolyte solutions, 0.01 M HCl and/or 0.1 M NaCl, are most representative of fasted human physiological conditions, owing to the pH and ionic strength conditions of the proximal gastrointestinal tract.

Electrolyte concentrations of 0.001 M HCl, 0.01 M HCl, and 0.1 M HCl correspond to pH 3, pH 2, and pH 1, respectively. Thus, a 0.01 M HCl solution simulates typical acidic conditions found in the stomach. A solution of 0.1 M NaCl provides a reasonable approximation of the ionic strength conditions found throughout the body, including the gastrointestinal fluids, although concentrations higher than 0.1 M may be employed to simulate fed conditions within the human GI tract.

Exemplary solutions of salts, acids, bases or combinations thereof, which exhibit the desired pH and ionic strength, include but are not limited to phosphoric acid/phosphate salts + sodium, potassium and calcium salts of chloride, acetic acid/acetate salts + sodium, potassium and calcium salts of chloride, carbonic acid/bicarbonate salts + sodium, potassium and calcium salts of chloride, and citric acid/citrate salts + sodium, potassium and calcium salts of chloride.

### 5. Bioadhesive Nanoparticulate Nystatin Compositions

Bioadhesive nanoparticulate nystatin compositions of the invention comprise at least one cationic surface stabilizer, which are described in more detail below. Bioadhesive formulations of nystatin exhibit exceptional bioadhesion to biological surfaces, such as mucous.

Bioadhesive formulations of the invention are primarily useful in cutaneous, oral, gastrointestinal, and vaginal candidiasis. In addition, such a formulation could be used in an inhaled version for pulmonary fungal infections. Non-bioadhesive nanoparticulate nystatin formulations may also be useful for systemic IV administration.

In the case of bioadhesive nanoparticulate nystatin compositions, the term "bioadhesion" is used to describe the adhesion between the nanoparticulate nystatin compositions and a biological substrate (*i.e.* gastrointestinal mucin, lung tissue, nasal mucosa, *etc.*). *See e.g.,* U.S. Patent No. 6,428,814 for "Bioadhesive Nanoparticulate Compositions Having Cationic Surface Stabilizers."

The bioadhesive nystatin compositions of the invention are useful in any situation in which it is desirable to apply the compositions to a biological surface. The bioadhesive nystatin compositions coat the targeted surface in a continuous and uniform film which is invisible to the naked human eye.

A bioadhesive nanoparticulate nystatin composition slows the transit of the composition, and some nystatin particles would also most likely adhere to tissue other than the mucous cells and therefore give a prolonged exposure to nystatin, thereby increasing absorption and the bioavailability of the administered dosage.

Preferred bioadhesive formulations of nanoparticulate nystatin comprise the cationic stabilizers 1,2 Dipalmitoyl-sn-Glycero-3-Phosphoethanolamine-N-[Amino(Polyethylene Glycol)2000] (sodium salt) (Avanti Polar Lipids, Alabaster, A1) (also known as DPPE-PEG(2000)-Amine Na); Poly(2-methacryloxyethyl trimethylammonium bromide) (Polysciences, Inc., Warrington, PA) (also known as S1001), or poloxamines such as Tetronic 908^{®}, also known as Poloxamine 908^{®}, which is a tetrafunctional block copolymer derived from sequential addition of propylene oxide and ethylene oxide to ethylenediamine (BASF Wyandotte Corporation, Parsippany. N.J.).

Other preferred useful bioadhesive surface stabilizers include lysozyme and long-chain polymers such as alginic acid, carrageenan, and POLYOX® (Dow, Midland, MI).

Useful surface stabilizers in the compositions of the invention can also bind to fungi, rather than to the biological surface being targeted. Such stabilizers include, but are not limited to, negatively charged surface stabilizers, such as lectins, sugars, antibodies or fragments thereof, *etc*.

### 6. Combination Pharmacokinetic Profile Compositions

In yet another embodiment of the invention, a first nanoparticulate nystatin composition providing a desired pharmacokinetic profile is co-administered, sequentially administered, or combined with at least one other nystatin composition that generates a desired different pharmacokinetic profile. More than two nystatin compositions can be co-administered, sequentially administered, or combined. While the first nystatin composition has a nanoparticulate particle size, the additional one or more nystatin compositions can be nanoparticulate, solubilized, or have a conventional microparticulate particle size.

For example, a first nystatin composition can have a nanoparticulate particle size, conferring a short Tₘₐₓ and typically a higher Cₘₐₓ. This first nystatin composition can be combined, co-administered, or sequentially administered with a second composition comprising: (1) nystatin having a larger (but still nanoparticulate as defined herein) particle size, and therefore exhibiting slower absorption, a longer T_{max,} and typically a lower Cₘₐₓ; or (2) a microparticulate or solubilized nystatin composition, exhibiting a longer T_{max,} and typically a lower Cₘₐₓ.

The second, third, fourth, *etc*., nystatin compositions can differ from the first, and from each other, for example: (1) in the effective average particle sizes of nystatin; or (2) in the dosage of nystatin. Such a combination composition can reduce the dose frequency required.

If the second nystatin composition has a nanoparticulate particle size, then preferably the nystatin particles of the second composition have at least one surface stabilizer associated with the surface of the drug particles. The one or more surface stabilizers can be the same as or different from the surface stabilizer(s) present in the first nystatin composition.

Preferably where co-administration of a "fast-acting" formulation and a "longer-lasting" formulation is desired, the two formulations are combined within a single composition, for example a dual-release composition.

### 7. Combination Active Agent Compositions

The invention encompasses the nanoparticulate nystatin compositions of the invention formulated or co-administered with one or more non-nystatin active agents, which are either conventional (solubilized or microparticulate) or nanoparticulate. Methods of using such combination compositions are also encompassed by the invention. The non-nystatin active agents can be present in a crystalline phase, an amorphous phase, a semi-crystalline phase, a semi-amorphous phase, or a mixture thereof.

The compound to be administered in combination with a nanoparticulate nystatin composition of the invention can be formulated separately from the nanoparticulate nystatin composition or co-formulated with the nanoparticulate nystatin composition. Where a nanoparticulate nystatin composition is co-formulated with a second active agent, the second active agent can be formulated in any suitable manner, such as immediate-release, rapid-onset, sustained-release, or dual-release form.

If the non-nystatin active agent has a nanoparticulate particle size *i.e.*, a particle size of less than about 2 microns, then preferably it will have one or more surface stabilizers associated with the surface of the active agent. In addition, if the active agent has a nanoparticulate particle size, then it is preferably poorly soluble and dispersible in at least one liquid dispersion media. By "poorly soluble" it is meant that the active agent has a solubility in a liquid dispersion media of less than about 30 mg/mL, less than about 20 mg/mL, less than about 10 mg/mL, or less than about 1 mg/mL. Useful liquid dispersion medias include, but are not limited to, water, aqueous salt solutions, safflower oil, and solvents such as ethanol, t-butanol, hexane, and glycol.

Such non-nystatin active agents can be, for example, a therapeutic agent. A therapeutic agent can be a pharmaceutical agent, including biologics. The active agent can be selected from a variety of known classes of drugs, including, for example, amino acids, proteins, peptides, nucleotides, anti-obesity drugs, central nervous system stimulants, carotenoids, corticosteroids, elastase inhibitors, anti-fungals, oncology therapies, anti-emetics, analgesics, cardiovascular agents, anti-inflammatory agents, such as NSAIDs and COX-2 inhibitors, anthelmintics, anti-arrhythmic agents, antibiotics (including penicillins), anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives (hypnotics and neuroleptics), astringents, alpha-adrenergic receptor blocking agents, beta-adrenoceptor blocking agents, blood products and substitutes, cardiac inotropic agents, contrast media, corticosteroids, cough suppressants (expectorants and mucolytics), diagnostic agents, diagnostic imaging agents, diuretics, dopaminergics (antiparkinsonian agents), haemostatics, immunological agents, lipid regulating agents, muscle relaxants, parasympathomimetics, parathyroid calcitonin and biphosphonates, prostaglandins, radio-pharmaceuticals, sex hormones (including steroids), anti-allergic agents, stimulants and anoretics, sympathomimetics, thyroid agents, vasodilators, and xanthines.

A description of these classes of active agents and a listing of species within each class can be found in Martindale's The Extra Pharmacopoeia. 31st Edition (The Pharmaceutical Press, London, 1996). The active agents are commercially available and/or can be prepared by techniques known in the art.

Exemplary nutraceuticals and dietary supplements are disclosed, for example, in Roberts et al., *Nutraceuticals: The Complete Encyclopedia of Supplements, Herbs, Vitamins, and Healing Foods* (American Nutraceutical Association, 2001). Dietary supplements and nutraceuticals are also disclosed in Physicians' Desk Reference for Nutritional Supplements, 1st Ed. (2001) and The Physicians' Desk Reference for Herbal Medicines, 1st Ed. (2001). A nutraceutical or dietary supplement, also known as a phytochemical or functional food, is generally any one of a class of dietary supplements, vitamins, minerals, herbs, or healing foods that have medical or pharmaceutical effects on the body.

Exemplary nutraceuticals or dietary supplements include, but are not limited to, lutein, folic acid, fatty acids (*e.g.*, DHA and ARA), fruit and vegetable extracts, vitamin and mineral supplements, phosphatidylserine, lipoic acid, melatonin, glucosamine/chondroitin, Aloe Vera, Guggul, glutamine, amino acids (*e.g.*, arginine, isoleucine, leucine, lysine, methionine, phenylanine, threonine, tryptophan, and valine), green tea, lycopene, whole foods, food additives, herbs, phytonutrients, antioxidants, flavonoid constituents of fruits, evening primrose oil, flax seeds, fish and marine animal oils, and probiotics. Nutraceuticals and dietary supplements also include bio-engineered foods genetically engineered to have a desired property, also known as "pharmafoods."

### 8. Sterile Filtered Nanoparticulate Nystatin Compositions

The nanoparticulate nystatin compositions of the invention can be sterile filtered. This obviates the need for heat sterilization, which can harm or degrade nystatin, as well as result in crystal growth and particle aggregation.

Sterile filtration can be difficult because of the required small particle size of the composition. Filtration is an effective method for sterilizing homogeneous solutions when the membrane filter pore size is less than or equal to about 0.2 microns (200 nm) because a 0.2 micron filter is sufficient to remove essentially all bacteria. Sterile filtration is normally not used to sterilize conventional suspensions of micron-sized nystatin because the nystatin particles are too large to pass through the membrane pores.

A sterile nanoparticulate nystatin dosage form is particularly useful in treating immunocompromised patients, infants or juvenile patients, and the elderly, as these patient groups are the most susceptible to infection caused by a non-sterile liquid dosage form.

Because the nanoparticulate nystatin compositions of the invention can be sterile filtered, and because the compositions can have a very small nystatin effective average particle size, the compositions are suitable for parenteral administration.

### 9. Miscellaneous Benefits of the Nanoparticulate Nystatin Compositions of the Invention

The nystatin composition may be formulated as a ready to use colloidal dispersion rather than as a suspension that may settle or must be prepared immediately before dosing. In addition, the bad taste associated with current conventional nystatin compositions may be reduced or eliminated. Finally, the nanoparticulate nystatin compositions do not require organic solvents or pH extremes.

### B. Compositions

The invention provides compositions comprising nanoparticulate nystatin particles and at least one surface stabilizer, as defined in the claims. The surface stabilizers are associated with the surface of the nystatin particles. Surface stabilizers useful herein do not chemically react with the nystatin particles or itself. Individual molecules of the surface stabilizer are essentially free of intermolecular cross-linkages.

The present invention also includes nanoparticulate nystatin compositions together with one or more non-toxic physiologically acceptable carriers, adjuvants, or vehicles, collectively referred to as carriers. The compositions can be formulated for parenteral injection (*e.g.*, intravenous, intramuscular, or subcutaneous), oral administration (in solid, liquid, or aerosol (*i.e.*, pulmonary) form), vaginal, nasal, rectal, ocular, local (powders, ointments or drops), buccal, intracisternal, intraperitoneal, or topical administration, and the like.

Exemplary types of formulations useful in the present invention include, but are not limited to, liquid dispersions, gels, aerosols, ointments, creams, solid dose forms, etc. ofnanoparticulate nystatin.

### 1. Nystatin Particles

As used herein the term nystatin refers to the poorly water-soluble antimycotic polyene antibiotic obtained from *Streptomyces noursei.* Nystatin is well known in the art and is readily recognized by one of ordinary skill.

Nystatin is an antifungal agent indicated for oral, gastrointestinal, and vaginal candidiasis. Oral candidiasis, in particular, is a common affliction of immunocompromised patients. Nystatin is indicated in the therapy of all infections caused by susceptible microorganisms in those patients in whom candidal (monilial) infections are most likely to complicate therapy.

The drug is useful in the treatment of patients receiving high and prolonged dosages of antibiotics, with or without corticosteroid therapy, elderly or debilitated patients, diabetics, patients receiving radiation therapy and antineoplastic drugs, and patients with prior or recurrent candidal infections. For example, nystatin is useful as an adjuvant preventive against secondary infections during antibiotic or corticosteroid therapy. In addition, nystatin is useful in clinical situations where the intestinal overgrowth of fungus may complicate the use of a broad-spectrum antibiotic, such as during prolonged or high-dosage antibiotic therapy, during the administration of corticosteroids or antimetabolites, or in the presence of debilitation or impaired host-defense mechanisms.

Nystatin is also indicated in the treatment of women with infections who are taking oral contraceptives or have histories of recurrent vaginal moniliasis. It is indicated for the treatment of cutaneous or mucocutaneous mycotic infections caused by *Candida albicans* and other suseptible *Candida* species.

Nystatin is indicated in the treatment of respiratory infections, such as pneumonia, bronchitis, tonsillitis, pharyngitis, laryngitis, and otitis media; genitourinary infections such as cystitis, pyelonephritis, urethritis, and gonorrhoea; and skin and soft tissue infections such as pyoderma, erysipelas, lymphangitis, and cellulitis.

### 2. Surface Stabilizers

The choice of a surface stabilizer for nystatin is non-trivial and required extensive experimentation to realize a desirable formulation. Accordingly, the present invention is directed to the surprising discovery that nystatin nanoparticulate compositions can be made.

Combinations of more than one surface stabilizer can be used in the invention, as defined in the claims. Useful surface stabilizers which can be employed include, but are not limited to, known organic and inorganic pharmaceutical excipients. Such excipients include various polymers, low molecular weight oligomers, natural products, and surfactants. Surface stabilizers include nonionic, cationic, zwitterionic, and ionic surfactants.

Preferred surface stabilizers include, but are not limited to: (1) Distearoyl Phosphatidyl Ethanolamine : polyethyleneglycol : NH₂ (DSPE-PEG-NH₂, which is a functionalized phospholipid; Avanti Polar Lipids, Alabaster, AL); (2) Poly (Lysine, tryptophan) (Sigma, St. Louis, MO); (3) Sodium Deoxycholate (Spectrum Quality Products); (4) polyvinylpyrrolidone (PVP); (5) Distearoyl Phosphatidyl Ethanolamine : polyethyleneglycol (DSPE-PEG) (Avanti Polar Lipids, Alabaster, AL); (6) povidone-iodine (Betadine); (7) Tetronic 908^{®}, also known as Poloxamine 908^{®}, which is a tetrafunctional block copolymer derived from sequential addition of propylene oxide and ethylene oxide to ethylenediamine (BASF Wyandotte Corporation, Parsippany, N.J.). (8) Poly(2-methacryloxyethyl trimethylammonium bromide) (also known as S1001) (Polysciences, Inc., Warrington, PA); and (9) Dipalmitoylphosphatidylethanolamine : polyethyleneglycol : NH₂ (also known as 1,2-Dipalmitoyl-sn-Glycero-3-Phosphoethanolamine-N-[Amino(Polyethylene Glycol)2000] and as DPPE-PEG-NH₂, Avanti Polar Lipids, Alabaster, AL).

Depending upon the desired method of administration, bioadhesive formulations of nanoparticulate nystatin can be prepared by selecting one or more surface stabilizers that impart bioadhesive properties to the resultant composition. Such surface stabilizers include, but are not limited to, cationic surface stabilizers, DPPE-PEG(2000)-Amine Na), S1001, poloxamines such as Tetronic 908^{®}, lysozyme, alginic acid, carrageenan (FMC Corp.), and POLYOX (Dow, Midland, MI). These surface stabilizers can also be used in combination with one or more non-bioadhesive surface stabilizers to form a bioadhesive formulation.

Representative examples of surface stabilizers include hydroxypropyl methylcellulose, hydroxypropylcellulose, polyvinylpyrrolidone, sodium lauryl sulfate, dioctylsulfosuccinate, gelatin, casein, lecithin (phosphatides), dextran, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers (*e.g.*, macrogol ethers such as cetomacrogol 1000), polyoxyethylene castor oil derivatives, polyoxyethylene sorbitan fatty acid esters (*e.g.*, the commercially available Tweens^{®} such as *e.g.*, Tween 20^{®} and Tween 80^{®} (ICI Speciality Chemicals)); polyethylene glycols (*e.g.*, Carbowaxs 3550^{®} and 934^{®} (Union Carbide)), polyoxyethylene stearates, colloidal silicon dioxide, phosphates, carboxymethylcellulose calcium, carboxymethylcellulose sodium, methylcellulose, hydroxyethylcellulose, hydroxypropylmethylcellulose phthalate, noncrystalline cellulose, magnesium aluminium silicate, triethanolamine, polyvinyl alcohol (PVA), 4-(1,1,3,3-tetramethylbutyl)-phenol polymer with ethylene oxide and formaldehyde (also known as tyloxapol, superione, and triton), poloxamers (*e.g.*, Pluronics F68^{®} and F108^{®}, which are block copolymers of ethylene oxide and propylene oxide); poloxamines (*e.g.*, Tetronic 908^{®}, also known as Poloxamine 908^{®}, which is a tetrafunctional block copolymer derived from sequential addition of propylene oxide and ethylene oxide to ethylenediamine (BASF Wyandotte Corporation, Parsippany, N.J.)); Tetronic 1508^{®} (T-1508) (BASF Wyandotte Corporation), Tritons X-200^{®}, which is an alkyl aryl polyether sulfonate (Rohm and Haas); Crodestas F-110^{®}, which is a mixture of sucrose stearate and sucrose distearate (Croda Inc.); p-isononylphenoxypoly-(glycidol), also known as Olin-1OG^{®} or Surfactant 10-G^{®} (Olin Chemicals, Stamford, CT); Crodestas SL-40^{®} (Croda, Inc.); and SA9OHCO, which is C₁₈H₃₇CH₂(CON(CH₃)-CH₂(CHOH)₄(CH₂0H)₂ (Eastman Kodak Co.); decanoyl-N-methylglucamide; n-decyl β-D-glucopyranoside; n-decyl β-D-maltopyranoside; n-dodecyl β- D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; PEG-phospholipid, PEG-cholesterol, PEG-cholesterol derivative, PEG-vitamin A, PEG-vitamin E, lysozyme, random copolymers of vinyl pyrrolidone and vinyl acetate, and the like.

Examples of useful cationic surface stabilizers include, but are not limited to, polymers, biopolymers, polysaccharides, cellulosics, alginates, phospholipids, and nonpolymeric compounds, such as zwitterionic stabilizers, poly-n-methylpyridinium, anthryul pyridinium chloride, cationic phospholipids, chitosan, polylysine, polyvinylimidazole, polybrene, polymethylmethacrylate trimethylammoniumbromide bromide (PMMTMABr), hexyldesyltrimethylammonium bromide (HDMAB), and polyvinylpyrrolidone-2-dimethylaminoethyl methacrylate dimethyl sulfate.

Other useful cationic stabilizers include, but are not limited to, cationic lipids, sulfonium, phosphonium, and quaternary ammonium compounds, such as stearyltrimethylammonium chloride, benzyl-di(2-chloroethyl)ethylammonium bromide, coconut trimethyl ammonium chloride or bromide, coconut methyl dihydroxyethyl ammonium chloride or bromide, decyl triethyl ammonium chloride, decyl dimethyl hydroxyethyl ammonium chloride or bromide, C₁₂₋₁₅dimethyl hydroxyethyl ammonium chloride or bromide, coconut dimethyl hydroxyethyl ammonium chloride or bromide, myristyl trimethyl ammonium methyl sulphate, lauryl dimethyl benzyl ammonium chloride or bromide, lauryl dimethyl (ethenoxy)₄ ammonium chloride or bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts and dialkyldimethylammonium salts, lauryl trimethyl ammonium chloride, ethoxylated alkyamidoalkyldialkylammonium salt and/or an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride and dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂, C₁₅, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride (ALIQUAT 336^{™}), POLYQUAT 10^{™}, tetrabutylammonium bromide, benzyl trimethylammonium bromide, choline esters (such as choline esters of fatty acids), benzalkonium chloride, stearalkonium chloride compounds (such as stearyltrimonium chloride and Distearyldimonium chloride), cetyl pyridinium bromide or chloride, halide salts of quaternized polyoxyethylalkylamines, MIRAPOL^{™} and ALKAQUAT^{™} (Alkaril Chemical Company), alkyl pyridinium salts; amines, such as alkylamines, dialkylamines, alkanolamines, polyethylenepolyamines, N,N-dialkylaminoalkyl acrylates, and vinyl pyridine, amine salts, such as lauryl amine acetate, stearyl amine acetate, alkylpyridinium salt, and alkylimidazolium salt, and amine oxides; imide azolinium salts; protonated quaternary acrylamides; methylated quaternary polymers, such as poly[diallyl dimethylammonium chloride] and poly-[N-methyl vinyl pyridinium chloride]; and cationic guar.

Such exemplary cationic surface stabilizers and other useful cationic surface stabilizers are described in J. Cross and E. Singer, Cationic Surfactants: Analytical and Biological Evaluation (Marcel Dekker, 1994); P. and D. Rubingh (Editor), Cationic Surfactants: Physical Chemistry (Marcel Dekker, 1991); and J. Richmond, Cationic Surfactants: Organic Chemistry, (Marcel Dekker, 1990).

Nonpolymeric surface stabilizers are any nonpolymeric compound, such as benzalkonium chloride, a carbonium compound, a phosphonium compound, an oxonium compound, a halonium compound, a cationic organometallic compound, a quarternary phosphorous compound, a pyridinium compound, an anilinium compound, an ammonium compound, a hydroxylammonium compound, a primary ammonium compound, a secondary ammonium compound, a tertiary ammonium compound, and quarternary ammonium compounds of the formula NR₁R₂R₃R₄⁽⁺⁾. For compounds of the formula NR₁R₂R₃R₄⁽⁺⁾:
(i) none of R₁-R₄ are CH₃;
(ii) one of R₁-R₄ is CH₃;
(iii) three of R₁-R₄ are CH₃;
(iv) all of R₁-R₄ are CH₃;
(v) two of R₁ -R₄ are CH₃, one of R₁-R₄ is C₆H₅CH₂, and one of R₁-R₄ is an alkyl chain of seven carbon atoms or less;
(vi) two of R₁-R₄ are CH₃, one of R₁-R₄ is C₆H₅CH₂, and one of R₁-R₄ is an alkyl chain of nineteen carbon atoms or more;
(vii) two of R₁ -R₄ are CH₃ and one of R₁-R₄ is the group C₆H₅(CH₂)ₙ, where n>1;
(viii) two of R₁-R₄ are CH₃, one of R₁-R₄ is C₆H₅CH₂, and one of R₁-R₄ comprises at least one heteroatom;
(ix) two of R₁-R₄ are CH₃, one of R₁-R₄ is C₆H₅CH₂, and one of R₁-R₄ comprises at least one halogen;
(x) two of R₁-R₄ are CH₃, one of R₁-R₄ is C₆H₅CH₂, and one of R₁-R₄ comprises at least one cyclic fragment;
(xi) two of R₁-R₄ are CH₃ and one of R₁-R₄ is a phenyl ring; or
(xii) two of R₁-R₄ are CH₃ and two of R₁-R₄ are purely aliphatic fragments.

Such compounds include, but are not limited to, behenalkonium chloride, benzethonium chloride, cetylpyridinium chloride, behentrimonium chloride, lauralkonium chloride, cetalkonium chloride, cetrimonium bromide, cetrimonium chloride, cethylamine hydrofluoride, chlorallylmethenamine chloride (Quatemium-15), distearyldimonium chloride (Quatemium-5), dodecyl dimethyl ethylbenzyl ammonium chloride(Quatemium-14), Quaternium-22, Quatemium-26, Quaternium-18 hectorite, dimethylaminoethylchloride hydrochloride, cysteine hydrochloride, diethanolammonium POE (10) oletyl ether phosphate, diethanolammonium POE (3)oleyl ether phosphate, tallow alkonium chloride, dimethyl dioctadecylammoniumbentonite, stearalkonium chloride, domiphen bromide, denatonium benzoate, myristalkonium chloride, laurtrimonium chloride, ethylenediamine dihydrochloride, guanidine hydrochloride, pyridoxine HCl, iofetamine hydrochloride, meglumine hydrochloride, methylbenzethonium chloride, myrtrimonium bromide, oleyltrimonium chloride, polyquaternium-1, procainehydrochloride, cocobetaine, stearalkonium bentonite, stearalkoniumhectonite, stearyl trihydroxyethyl propylenediamine dihydrofluoride, tallowtrimonium chloride, and hexadecyltrimethyl ammonium bromide.

Most of these surface stabilizers are known pharmaceutical excipients and are described in detail in the Handbook of Pharmaceutical Excipients, published jointly by the American Pharmaceutical Association and The Pharmaceutical Society of Great Britain (The Pharmaceutical Press, 2000).

The surface stabilizers are commercially available and/or can be prepared by techniques known in the art.

### 3. Other Pharmaceutical Excipients

Pharmaceutical compositions according to the invention may also comprise one or more binding agents, filling agents, lubricating agents, suspending agents, sweeteners, flavoring agents, preservatives, buffers, wetting agents, disintegrants, effervescent agents, and other excipients. Such excipients are known in the art.

Examples of filling agents are lactose monohydrate, lactose anhydrous, and various starches; examples of binding agents are various celluloses and cross-linked polyvinylpyrrolidone, microcrystalline cellulose, such as Avicel^{®} PH101 and Avicel^{®} PH102, microcrystalline cellulose, and silicified microcrystalline cellulose (ProSolv SMCC™).

Suitable lubricants, including agents that act on the flowability of the powder to be compressed, are colloidal silicon dioxide, such as Aerosil^{®} 200, talc, stearic acid, magnesium stearate, calcium stearate, and silica gel.

Examples of sweeteners are any natural or artificial sweetener, such as sucrose, xylitol, sodium saccharin, cyclamate, aspartame, and acsulfame. Examples of flavoring agents are Magnasweet^{®} (trademark of MAFCO), bubble gum flavor, and fruit flavors, and the like.

Examples of preservatives are potassium sorbate, methylparaben, propylparaben, benzoic acid and its salts, other esters of parahydroxybenzoic acid such as butylparaben, alcohols such as ethyl or benzyl alcohol, phenolic compounds such as phenol, or quarternary compounds such as benzalkonium chloride.

Suitable diluents include pharmaceutically acceptable inert fillers, such as microcrystalline cellulose, lactose, dibasic calcium phosphate, saccharides, and/or mixtures of any of the foregoing. Examples of diluents include microcrystalline cellulose, such as Avicel^{®} PH101 and Avicel^{®} PH102; lactose such as lactose monohydrate, lactose anhydrous, and Pharmatose^{®} DCL21; dibasic calcium phosphate such as Emcompress^{®}; mannitol; starch; sorbitol; sucrose; and glucose.

Suitable disintegrants include lightly crosslinked polyvinyl pyrrolidone, corn starch, potato starch, maize starch, and modified starches, croscarmellose sodium, cross-povidone, sodium starch glycolate, and mixtures thereof.

Examples of effervescent agents are effervescent couples such as an organic acid and a carbonate or bicarbonate. Suitable organic acids include, for example, citric, tartaric, malic, fumaric, adipic, succinic, and alginic acids and anhydrides and acid salts. Suitable carbonates and bicarbonates include, for example, sodium carbonate, sodium bicarbonate, potassium carbonate, potassium bicarbonate, magnesium carbonate, sodium glycine carbonate, L-lysine carbonate, and arginine carbonate. Alternatively, only the sodium bicarbonate component of the effervescent couple may be present.

### 4. Nanoparticulate Nystatin Particle Size

As used herein, particle size is determined on the basis of the weight average particle size as measured by conventional particle size measuring techniques well known to those skilled in the art. Such techniques include, for example, sedimentation field flow fractionation, photon correlation spectroscopy, light scattering, and disk centrifugation.

The compositions of the invention contain nystatin nanoparticles which have an effective average particle size of less than about 2000 nm (i.e., 2 microns), less than about 1900 nm, less than less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, or less than about 50 nm, as measured by light-scattering methods, microscopy, or other appropriate methods.

If the nanoparticulate nystatin composition additionally comprises one or more non-nystatin nanoparticulate active agents, then such active agents have an effective average particle size of less than about 2000 nm (*i.e.,* 2 microns). In other embodiments of the invention, the nanoparticulate non-nystatin active agents can have an effective average particle size of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 run, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, or less than about 50 nm, as measured by the above-noted techniques.

By "an effective average particle size of less than about 2000 nm" it is meant that at least 50% by weight of the nanoparticulate nystatin particles or nanoparticulate non-nystatin active agent is present in particles having a size of less than about 2000 nm when measured by the above-noted techniques. In other embodiments of the invention, at least about 70%, at least about 90%, or at least about 95% of the nanoparticulate nystatin particles nanoparticulate non-nystatin active agent particles have a particle size of less than about 2000 nm.

If the nanoparticulate nystatin composition is combined with a conventional or microparticulate nystatin composition or non-nystatin active agent composition, then such a composition is either solubilized or has an effective average particle size of greater than about 2 microns. By "an effective average particle size of greater than about 2 microns" it is meant that at least 50% of the conventional nystatin or conventional non-nystatin active agent particles have a particle size of greater than about 2 microns, by weight, when measured by the above-noted techniques. In other embodiments of the invention, at least about 70%, about 90%, about 95%, or about 99% of the conventional nystatin or conventional non-nystatin active agent particles have a particle size greater than about 2 microns.

### 5. Concentration of Nanoparticulate Nystatin and Surface Stabilizers

The relative amounts of nystatin and one or more surface stabilizers can vary widely. The optimal amount of the individual components can depend, for example, upon the particular active agent selected, the hydrophilic lipophilic balance (HLB), melting point, and the surface tension of water solutions of the stabilizer, *etc*.

The concentration of nystatin can vary from about 99.5% to about 0.001%, from about 95% to about 0.1%, and from about 90% to about 0.5%, by weight, based on the total combined weight of the nystatin and at least one surface stabilizer, not including other excipients.

The concentration of the at least one surface stabilizer can vary from about 0.5% to about 99.999%, from about 5.0% to about 99.9%, or from about 10.0% to about 99.5%, by weight, based on the total combined dry weight of the nystatin and at least one surface stabilizer, not including other excipients.

### B. Methods of Making Nanoparticulate Formulations

The nanoparticulate nystatin compositions can be made using, for example, milling, homogenization, or precipitation techniques. Exemplary methods of making nanoparticulate compositions are described in the'684 patent. Methods of making nanoparticulate compositions are also described in U.S. Patent No. 5,518,187 for "Method of Grinding Pharmaceutical Substances;" U.S. Patent No. 5,718,388 for "Continuous Method of Grinding Pharmaceutical Substances;" U.S. Patent No. 5,862,999 for "Method of Grinding Pharmaceutical Substances;" U.S. Patent No. 5,665,331 for "Co-Microprecipitation of Nanoparticulate Pharmaceutical Agents with Crystal Growth Modifiers;" U.S. Patent No. 5,662,883 for "Co-Microprecipitation of Nanoparticulate Pharmaceutical Agents with Crystal Growth Modifiers;" U.S. Patent No. 5,560,932 for "Microprecipitation of Nanoparticulate Pharmaceutical Agents;" U.S. Patent No. 5,543,133 for "Process of Preparing X-Ray Contrast Compositions Containing Nanoparticles;" U.S. Patent No. 5,534,270 for "Method of Preparing Stable Drug Nanoparticles;" U.S. Patent No. 5,510,118 for "Process of Preparing Therapeutic Compositions Containing Nanoparticles;" and U.S. Patent No. 5,470,583 for "Method of Preparing Nanoparticle Compositions Containing Charged Phospholipids to Reduce Aggregation."

Nanoparticulate nystatin compositions made, *e.g*., via milling, homogenization, or precipitation, can be utilized in solid or liquid dosage formulations, such as controlled release formulations, solid dose fast melt formulations, aerosol formulations, lyophilized formulations, tablets, capsules, solid lozenge, powders, etc.

### 1. Milling to Obtain Nanoparticulate Nystatin Dispersions

Milling nystatin to obtain a nanoparticulate dispersion comprises dispersing nystatin particles in a liquid dispersion medium in which nystatin is poorly soluble, followed by applying mechanical means in the presence of grinding media to reduce the particle size of nystatin to the desired effective average particle size. The dispersion medium can be, for example, water, safflower oil, ethanol, t-butanol, glycerin, polyethylene glycol (PEG), hexane, or glycol.

The nystatin particles can be reduced in size in the presence of at least one surface stabilizer. Alternatively, the nystatin particles can be contacted with one or more surface stabilizers after attrition. Other compounds, such as a diluent, can be added to the nystatin/surface stabilizer composition during the size reduction process. Dispersions can be manufactured continuously or in a batch mode.

### 2. Precipitation to Obtain Nanoparticulate Nystatin Compositions

Another method of forming the desired nanoparticulate nystatin composition is by microprecipitation. This is a method of preparing stable dispersions of poorly soluble active agents in the presence of one or more surface stabilizers and one or more colloid stability enhancing surface active agents free of any trace toxic solvents or solubilized heavy metal impurities. Such a method comprises, for example: (1) dissolving nystatin in a suitable solvent; (2) adding the formulation from step (1) to a solution comprising at least one surface stabilizer; and (3) precipitating the formulation from step (2) using an appropriate non-solvent. The method can be followed by removal of any formed salt, if present, by dialysis or diafiltration and concentration of the dispersion by conventional means.

### 3. Homogenization to Obtain Nystatin Nanoparticulate Compositions

Exemplary homogenization methods of preparing active agent nanoparticulate compositions are described in U.S. Patent No. 5,510,118, for "Process of Preparing Therapeutic Compositions Containing Nanoparticles."

Such a method comprises dispersing nystatin particles in a liquid dispersion medium, followed by subjecting the dispersion to homogenization to reduce the particle size of the nystatin to the desired effective average particle size. The nystatin particles can be reduced in size in the presence of at least one surface stabilizer. Alternatively, the nystatin particles can be contacted with one or more surface stabilizers either before or after attrition. Other compounds, such as a diluent, can be added to the nystatin/surface stabilizer composition either before, during, or after the size reduction process. Dispersions can be manufactured continuously or in a batch mode.

### C. Methods of Using Nystatin Formulations of the Current Invention

The nystatin compositions of the present invention can be administered to a subject via any conventional means including, but not limited to, orally, rectally, ocularly, parenterally (*e.g*., intravenous, intramuscular, or subcutaneous), intracisternally, pulmonary, intravaginally, intraperitoneally, locally (*e.g.*, powders, ointments or drops), or as a buccal or nasal spray. As used herein, the term "subject" is used to mean an animal, preferably a mammal, including a human or non-human. The terms patient and subject may be used interchangeably.

The method of the invention comprises administering to a subject an effective amount of a composition comprising nystatin.

The compositions of the invention are useful in treating infections of susceptible organisms. Oral candidiasis, in particular, is a common affliction of immunocompromised patients. Nystatin is indicated in the therapy of all infections caused by susceptible microorganisms in those patients in whom candidal (monilial) infections are most likely to complicate therapy.

Nystatin is useful in the treatment of patients receiving high and prolonged dosages of antibiotics, with or without corticosteroid therapy, elderly or debilitated patients, diabetics, patients receiving radiation therapy and antineoplastic drugs, and patients with prior or recurrent candidal infections. For example, nystatin is useful as an adjuvant preventive against secondary infections during antibiotic or corticosteroid therapy. In addition, nystatin is useful in clinical situations where the intestinal overgrowth of fungus may complicate the use of a broad-spectrum antibiotic, such as during prolonged or high-dosage antibiotic therapy, during the administration of corticosteroids or antimetabolites, or in the presence of debilitation or impaired host-defense mechanisms.

It is also indicated in the treatment of women with infections who are taking oral contraceptives or have histories of recurrent vaginal moniliasis. It is indicated for the treatment of cutaneous or mucocutaneous mycotic infections caused by *Candida albicans* and other suseptible *Candida* species.

Nystatin is indicated in the treatment of respiratory infections, such as pneumonia, bronchitis, tonsillitis, pharyngitis, laryngitis, and otitis media; genitourinary infections such as cystitis, pyelonephritis, urethritis, and gonorrhoea; and skin and soft tissue infections such as pyoderma, erysipelas, lymphangitis, and cellulitis.

Compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents, or vehicles including water, ethanol, polyols (propyleneglycol, polyethylene-glycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions, and by the use of surfactants.

The nanoparticulate compositions may also contain adjuvants such as preserving, wetting, emulsifying, and dispensing agents. Prevention of the growth of microorganisms can be ensured by various antibacterial and antifungal agents, such as parabens, chlorobutanol, phenol, sorbic acid, and the like. It may also be desirable to include isotonic agents, such as sugars, sodium chloride, and the like. Prolonged absorption of the injectable pharmaceutical form can be brought about by the use of agents delaying absorption, such as aluminum monostearate and gelatin.

Solid dosage forms for oral administration include, but are not limited to, capsules, tablets, pills, powders, and granules. In such solid dosage forms, the active agent is admixed with at least one of the following: (a) one or more inert excipients (or carriers), such as sodium citrate or dicalcium phosphate; (b) fillers or extenders, such as starches, lactose, sucrose, glucose, mannitol, and silicic acid; (c) binders, such as carboxymethylcellulose, alignates, gelatin, polyvinylpyrrolidone, sucrose, and acacia; (d) humectants, such as glycerol; (e) disintegrating agents, such as agar-agar, calcium carbonate, potato or tapioca starch, alginic acid, certain complex silicates, and sodium carbonate; (f) solution retarders, such as paraffin; (g) absorption accelerators, such as quaternary ammonium compounds; (h) wetting agents, such as cetyl alcohol and glycerol monostearate; (i) adsorbents, such as kaolin and bentonite; and (j) lubricants, such as talc, calcium stearate, magnesium stearate, solid polyethylene glycols, sodium lauryl sulfate, or mixtures thereof. For capsules, tablets, and pills, the dosage forms may also comprise buffering agents.

Liquid dosage forms for oral administration include pharmaceutically acceptable emulsions, solutions, suspensions, syrups, and elixirs. In addition to the active agent, the liquid dosage forms may comprise inert diluents commonly used in the art, such as water or other solvents, solubilizing agents, and emulsifiers. Exemplary emulsifiers are ethyl alcohol, isopropyl alcohol, ethyl carbonate, ethyl acetate, benzyl alcohol, benzyl benzoate, propyleneglycol, 1,3-butyleneglycol, dimethylformamide, oils, such as cottonseed oil, groundnut oil, corn germ oil, olive oil, castor oil, and sesame oil, glycerol, tetrahydrofurfuryl alcohol, polyethyleneglycols, fatty acid esters of sorbitan, or mixtures of these substances, and the like.

Besides such inert diluents, the composition can also include adjuvants, such as wetting agents, emulsifying and suspending agents, sweetening, flavoring, and perfuming agents.

One of ordinary skill will appreciate that effective amounts of nystatin can be determined empirically and can be employed in pure form or, where such forms exist, in pharmaceutically acceptable salt, ester, or prodrug form. Actual dosage levels of nystatin in the nanoparticulate compositions of the invention may be varied to obtain an amount of nystatin that is effective to obtain a desired therapeutic response for a particular composition and method of administration. The selected dosage level therefore depends upon the desired therapeutic effect, the route of administration, the potency of the administered nystatin, the desired duration of treatment, and other factors.

Dosage unit compositions may contain such amounts of such submultiples thereof as may be used to make up the daily dose. It will be understood, however, that the specific dose level for any particular patient will depend upon a variety of factors: the type and degree of the cellular or physiological response to be achieved; activity of the specific agent or composition employed; the specific agents or composition employed; the age, body weight, general health, sex, and diet of the patient; the time of administration, route of administration, and rate of excretion of the agent; the duration of the treatment; drugs used in combination or coincidental with the specific agent; and like factors well known in the medical arts.

The following examples are given to illustrate the present invention. It should be understood, however, that the invention is not to be limited to the specific conditions or details described in these examples.

In the examples that follow, the value for D50 is the particle size below which 50% of the active agent particles fail. Similarly, D90 is the particle size below which 90% of the active agent particles fall.

The formulations in the examples that follow were also investigated using a light microscope. Here, "stable" nanoparticulate dispersions (uniform Brownian motion) were readily distinguishable from "aggregated" dispersions (relatively large, nonuniform particles without motion).

### Example 1

The purpose of this example was to prepare nanoparticulate dispersions of nystatin.

A wet-milling technique was used to produce aqueous nanoparticle dispersions of nystatin stabilized by polymeric surface stabilizers. Stabilizers were selected for their ability to promote particle interaction with biological surfaces via charge and chain-entanglement interactions.

Three formulations were made, summarized in Table 1, below, by ball-milling the drug and surface stabilizer using a roller mill (U.S. Stoneware) and 0.8 mm YSZ Milling Media (Tosoh) for 24 hours. Particle size of the resultant compositions was analyzed by microscopy and static light scattering using Horiba LA-910 Laser Scattering Particle Size Distribution Analyzer (Horiba Instruments, Irvine, CA).

| **TABLE 1** | | | | |
|---|---|---|---|---|
| **Nystatin Compositions** | | | | |
| **Formulation** | **Drug** | **Surface Stabilizer** | **Mean Particle Size (nm)** | **D90 (nm)** |
| 1 | 1% Nystatin | 0.2% DPPE-PEG(2000)-Amine Na | 304 | 440 |
| 2 | 1% Nystatin | 0.5% S1001 | 1,290 | 3,676 |
| 3 | 1% Nystatin | 0.5% Tetronic 908^{®} | 190 | 353 |

**Results**. Wet-milling successfully reduced nystatin particle size, producing stable nanoparticulate dispersions characterized by a mean particle size of less than 2 µm.

In conclusion, nystatin is amenable to formulation by a nanoparticulate technique that may offer enhanced therapeutic efficacy against candidiasis compared to currently-available formulations. The particulates are expected to demonstrate affinity for biological surfaces, and all formulations are expected to be successful in reducing levels of candida, demonstrating that drug activity is retained through the milling process.

### Example 2

The purpose of this example was to prepare nanoparticulate nystatin formulations having various novel surface stabilizers, which were selected based on their potential bioadhesive or antimicrobial properties.

The four nystatin formulations prepared are described in Table 2.

**TABLE 2**

| **Formulation** | **Drug Concentration (nominal, by weight)** | **Stabilizer and Concentration (nominal, by weight)** | **Stabilizer Property** |
|---|---|---|---|
| 1 | 5% Nystatin | 1.25% Sodium Deoxycholate | Used as solubilizer in polyene formulations |
| 2 | 5% Nystatin | 1% DSPE-PEG(2000)-Amine | Cationic phospholipid-PEG |
| 3 | 2% Nystatin | 1% Poly(Lysine, Tryptophan) 4:1 Hydrobromide, MW (38,000) | Cationic randomco-polyamino acid |
| 4 | 5% Nystatin | 1% Povidone-iodine (Betadine) | Antimicrobial activity |

The formulations were milled using either high-energy (Nanomill™(*see e.g.,* U.S. Patent No. 6,431,478 for "Small Scale Mill"), 10 ml chamber) or low energy (ball milling) techniques. Milling was stopped when 90% of the particles (by weight) were less than 400 nm in diameter. Particle size was determined by static light scattering using a Horiba LA-910 light-scattering particle size analyzer (Horiba Instruments, Irvine, CA). Zeta potential was measured by electrophoresis in 5x10⁻⁴ M NaCl (Malvem ZetaSizer). Dispersibility was verified by phase contrast microscopy.

### Results:

The results of characterization are summarized in Table 3.

**TABLE 3**

| **Formulation** | **Milling Method** | **Milling Time** | **Mean Particle Size (nm)** | **D90 (nm)** | **pH** | **Zeta Potential (mV)*** |
|---|---|---|---|---|---|---|
| 1 | Nanomill^{™}, 5500 rpm | 30 min | 138 | 218 | 7.2 | -28.7 |
| 2 | Ball Mill | 3 Days | 213 | 319 | 5.3 | 0.7 |
| 3 | Ball Mill | 1 Day | 149 | 270 | 6.8 | 47.7 |
| 4 | Nanomill^{™}, 2500 rpm | 60 min | 177 | 331 | 5.9 | -27.0 |

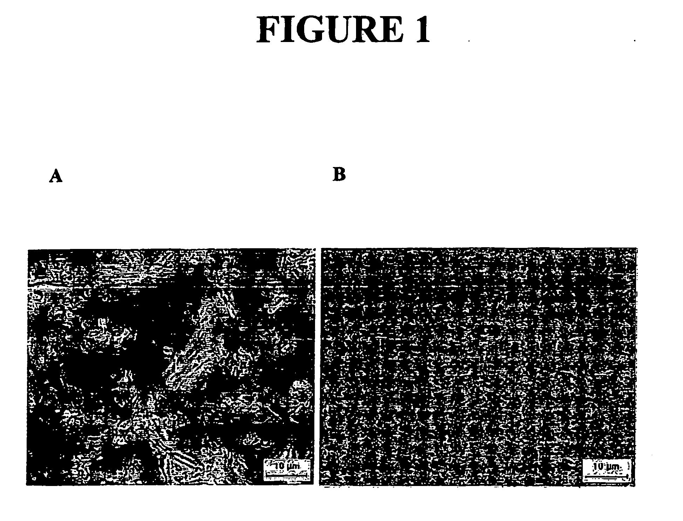

Figure 1 shows representative photomicrographs of the nystatin crystals before (Fig. 1A) and after (Fig. 1B) milling.

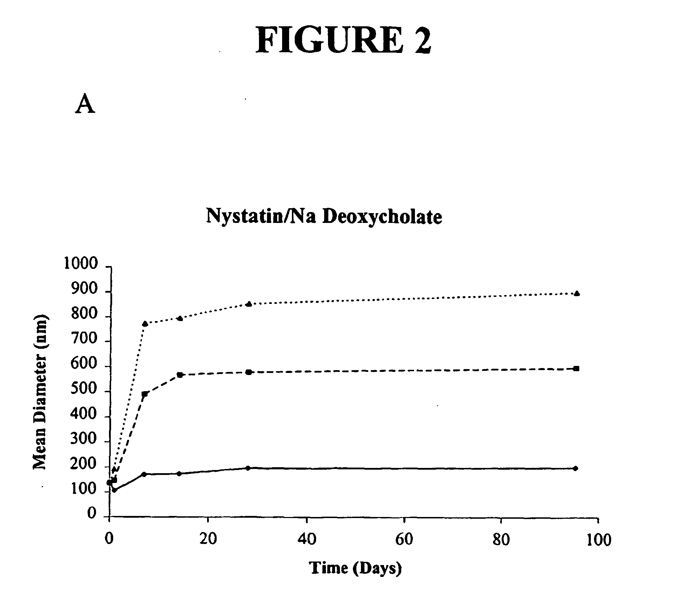

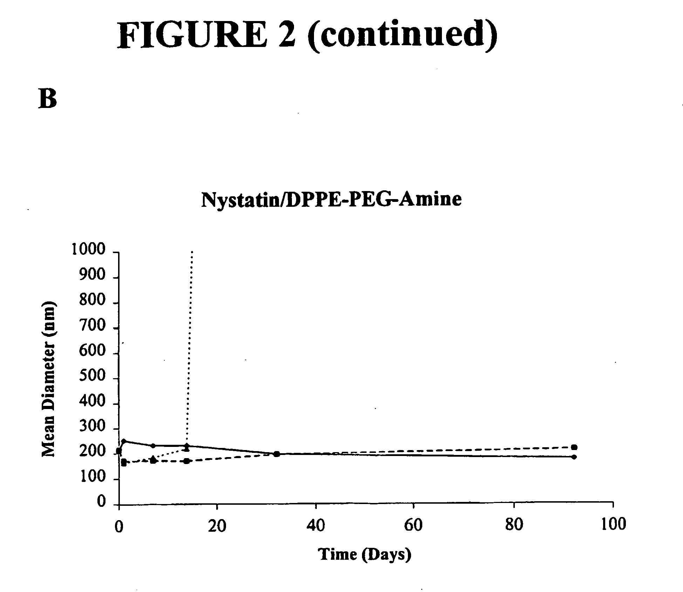

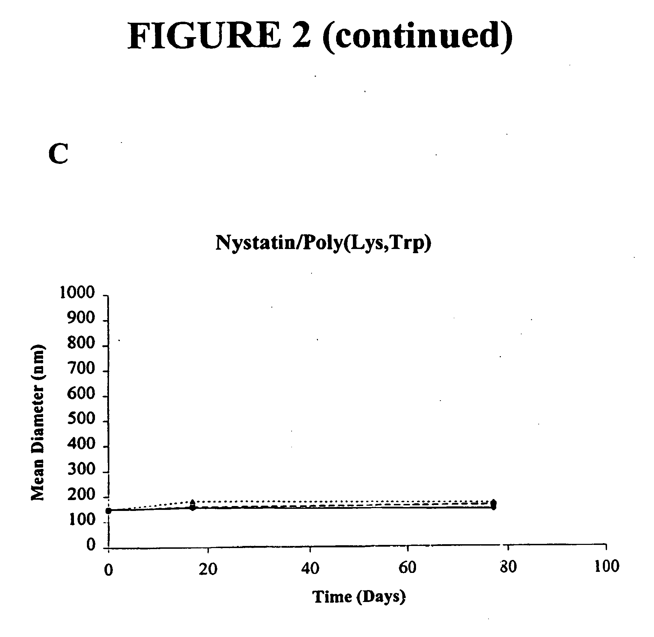

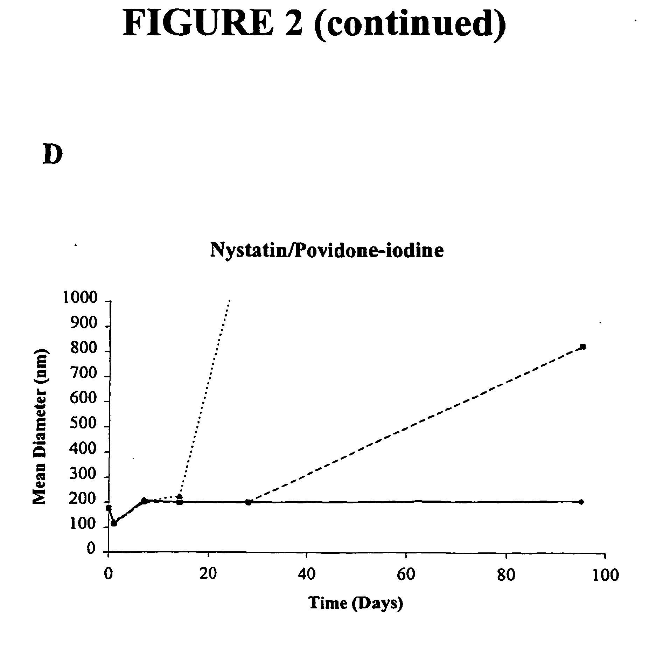

Particle size stability under controlled conditions was monitored over time at Shows the results of monitoring the particle size stability over time at 5°C (solid line), 25°C (dashed line), and 40°C (dotted line) for each formulation. The results are summarized in the graphs of Figures 2(A), (B), (C), and (D).

These results demonstrate that nystatin can be successfully formulated into nanoparticulate compositions using novel stabilizers that may confer additional therapeutic advantage to the final formulation. All four formulations exhibited mean particle sizes of less than 250 nm and were free of agglomeration. Physical stability as measured by particle size was acceptable for all four formulations at 5°C. Notably, the formulation stabilized by Poly(Lys,Trp) exhibited virtually no particle size growth at all three temperatures.

### Example 3

The purpose of this example was to determine whether a cationic surface charge, such as that obtained with the use of a cationic surface stabilizer in nanoparticulate nystatin formulations, enhances the adhesion of small particles to cells.

Cell-binding experiments were performed with polystyrene latex microspheres as a model. A positive surface charge would be expected to enhance the interaction of particles with cell-surface macromolecules, which have a net negative charge.

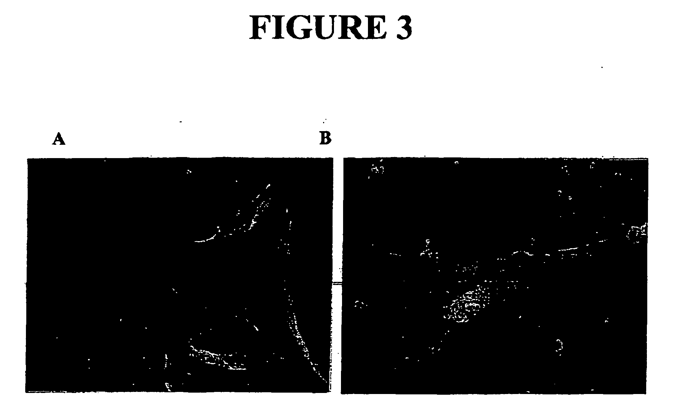

Cationic microspheres with a mean zeta-potential (51.5 mV) comparable to nystatin Formulation 3 of Example 2 were tested against anionic microspheres (mean zeta-potential = -50.9 mV). The microspheres were incubated with NIH/3T3 fibroblasts, washed thoroughly, fixed, and subjected to SEM analysis. Figure 3 shows representative micrographs of cells with anionic particles (Fig. 3A) and cationic particles (Fig. 3B).

The results indicate that positively-charged particles interact more strongly with the cell surface than negatively-charged particles, and it is believed that nanoparticulate nystatin formulations with comparable zeta potentials will follow the same trend.

### Example 4

The purpose of this example was to determine if milling of nystatin affects the drug's activity.

The minimum inhibitory concentration (MIC) of five milled nystatin compositions was compared to the MIC of two unmilled nystatin compositions. Nystatin for the milled nanoparticulate compositions was obtained from Sigma-Aldrich Co. and the two unmilled nystatin compositions were obtained from Sigma-Aldrich Co. and Paddock Laboratories, Inc. Details regarding the milled and unmilled nystatin formulations are given in Table 7 below, including particle size of the milled nanoparticulate nystatin compositions, the identify of the surface stabilizer for the milled nanoparticulate nystatin compositions, and the potency (USP U/ml) and MIC for each nystatin composition.

| **TABLE 7** | | | | |
|---|---|---|---|---|
| **Nystatin Concentration** | **Surface Stabilizer and Concentration** | **Mean Particle Size (nm)** | **Potency (USP U/ml)** | **MIC** |
| 5% (Sigma) | DSPE-PEG-NH₂¹ | 192 | 253,000 | 1:100,000 |
| 2% (Sigma) | Poly(Lys, Trp)² | 129 | 101,200 | 1:10,000 |
| 5% (Sigma) | PVP-1 | 218 | 253,000 | 1:100,000 |
| 5% (Sigma) | Na Deoxycholate⁴ | 152 | 253,000 | 1:10,000 |
| 5% (Sigma) | DSPE-PEG⁵ | 135 | 253,000 | 1:100,000 |
| | | | | |
| 5% (Sigma) | N/A - unmilled | N/A | 253,000 | 1:10,000 |
| 4% (Paddock) | N/A - unmilled | N/A | 253,000 | 1:100,000 |

| | | | | |
|---|---|---|---|---|
| ¹DSPE-PEG-NH₂ (also known as DSPE-PEG(2000)Amine, DSPE-PEG(2000)NH₂, DSPE-PEG(2000)NH₂ Na) is 1,2-Distearoyl-sn-Glycero-3-Phosphoethanolamine-N-[Amino(Polyethylene Glycol)2000], is a cationic phospholipid-PEG available from Avanti Polar Lipids (Alabaster, AL). ²Poly(Lysine, Tryptophan), which is a cationic random co-polyamino acid, is available from Sigma (St. Louris, MO). ⁴Na Deoxycholate (sodium deoxycholate) is used as a solubilizer in polyene formulations and is available from Spectrum Quality Products. ⁵DSPE-PEG is available from Avanti Polar Lipids (Alabaster, AL). | | | | |

The minimum inhibitory concentration (MIC) of the five milled nystatin formulations, one unmilled sample, and one sample of a marketed formulation, were determined in cultures of *C*. *albicans.* MIC as reported here is the maximum dilution of formulation in culture broth which inhibits growth of *C albicans.* As shown in Table 7, above, with the exception of the Na Deoxycholate-stabilized sample of nanoparticulate nystatin, none of the milled formulations exhibited any significant differences in MIC, and surprisingly, were more active than unmilled nystatin material. Activity as measured by MIC was comparable to that of the marketed product. These data confirm that the milling process does not decrease the activity of nystatin.

## Claims

1. A stable nystatin nanoparticulate composition comprising:
(a) particles of nystatin or a salt thereof; and
(b) associated with or adsorbed to the surface thereof at least one surface stabilizer selected from the group consisting of the sodium salt of 1,2-dipalmitoyl-sn-glycero-3-phosphoethanolamine-N-[amino(polyethyleneglycol)2000]; poly(2-methacryloxyethyl trimethylammonium bromide); a tetrafunctional block copolymer derived from sequential addition of propylene oxide and ethylene oxide to ethylenediamine; sodium deoxycholate; poly(lysine, tryptophan) hydrobromide; povidone-iodine; polyvinylpyrrolidone; distearoyl phosphatidyl ethanolamine : polyethyleneglycol : NH₂; and distearoyl phosphatidyl ethanolamine : polyethyleneglycol,
wherein the nystatin particles have an effective average particle size of less than 2000 nm.

2. The composition of claim 1 formulated into a liquid dosage form, wherein the dosage form has a viscosity of less than about 2000 mPa·s, measured at 20°C, at a shear rate of 0.1 (1/s).

3. The composition of claim 2, having a viscosity at a shear rate of 0.1 (l/s) selected from the group consisting of from about 2000 mPa·s to about 1 mPa·s, from about 1900 mPa·s to about 1 mPa·s, from about 1800 mPa·s to about 1 mPa·s, from about 1700 mPa·s to about 1 mPa·s, from about 1600 mPa·s to about 1 mPa·s, from about 1500 mPa·s to about 1 mPa·s, from about 1400 mPa·s to about 1 mPa·s, from about 1300 mPa·s to about 1 mPa·s, from about 1200 mPa·s to about 1 mPa·s, from about 1100 mPa·s to about 1 mPa·s, from about 1000 mPa·s to about 1 mPa·s, from about 900 mPa·s to about 1 mPa·s, from about 800 mPa·s to about 1 mPa·s, from about 700 mPa·s to about 1 mPa·s, from about 600 mPa·s to about 1 mPa·s, from about 500 mPa·s to about 1 mPa·s, from about 400 mPa·s to about 1 mPa·s, from about 300 mPa·s to about 1 mPa·s, from about 200 mPa·s to about 1 mPa·s, from about 175 mPa·s to about 1 mPa·s, from about 150 mPa·s to about 1 mPa·s, from about 125 mPa·s to about 1 mPa·s, from about 100 mPa·s to about 1 mPa·s, from about 75 mPa·s to about 1 mPa·s, from about 50 mPa·s to about 1 mPa·s, from about 25 mPa·s to about 1 mPa·s, from about 15 mPa·s to about 1 mPa·s, from about 10 mPa·s to about 1 mPa·s, and from about 5 mPa·s to.about 1 mPa·s.

4. The composition of either of claims 2 and 3, wherein the viscosity of the dosage form is selected from the group consisting of less than about 1/200, less than about 1/100, less than about 1/50, less than about 1/25, and less than about 1/10 of the viscosity of a nystatin composition wherein the active agent is present in solubilised form or present as particles having an effective average particle size of greater than two microns, at about the same concentration per ml of nystatin and/or wherein the viscosity of the dosage form is selected from the group consisting of less than about 5%, less than about 10%, less than about 15%, less than about 20%, less than about 25%, less than about 30%, less than about 35%, less than about 40%, less than about 45%, less than about 50%, less than about 55%, less than about 60%, less than about 65%, less than about 70%, less than about 75%, less than about 80%, less than about 85%, and less than about 90% of the viscosity of a nystatin composition, wherein the active agent is present in solubilised form or present as particles having an effective average particle size of greater than two microns at about the same concentration per ml of nystatin.

5. The composition of any one of claims 1-4, wherein the nystatin is selected from the group consisting of a crystalline phase, an amorphous phase, a semi-crystalline phase, a semi-amorphous phase, and mixtures thereof.

6. The composition of any one of claims 1-5, wherein the effective average particle size of the nanoparticulate nystatin particules is selected from the group consisting of less than about 1500 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 100 nm, less than about 75 nm, and less than about 50 nm.

7. The composition of any one of claims 1-6, wherein:
(a) the nystatin is present in an amount selected from the group consisting of from about 99.5% to about 0.001%, from about 95% to about 0.1%, and from about 90% to about 0.5%, by weight, based on the total combined weight of the nystatin and at least one surface stabilizer, not including other excipients; and/or
(b) at least one surface stabilizer is present in an amount selected from the group consisting of from about 0.5% to about 99.999% by weight, from about 5.0% to about 99.9% by weight, and from about 10.0% to about 99.5% by weight, based on the total combined dry weight of the nystatin and at least one surface stabilizer, not including other excipients;
or wherein the composition comprises at least two surface stabilizers.

8. The composition of any one of claims 1 to 7, wherein the composition is bioadhesive.

9. The composition of any one of claims 1 to 8, wherein the composition is formulated into a liquid dosage form, and the amount of nystatin per ml is equal to or greater than the amount of nystatin per ml of a nystatin composition wherein the active agent is present in solubilised form or present as particles having an effective average particle size of greater than two microns.

10. The composition of any one of claims 1 to 9, wherein the composition further comprises a nystatin composition having an effective average particle size of greater than about 2 microns.

11. The composition of any one of claims 1 to 10, wherein the composition additionally comprises at least one additional nanoparticulate nystatin composition having an effective average particle size of less than about 2 microns,
wherein said additional nanoparticulate nystatin composition has an effective average particle size which is different than the effective average particle size of the nanoparticulate nystatin composition of claims 1 to 10.

12. The composition of any one of claims 1 to 11, additionally comprising at least one non-nystatin active agent, particularly selected from the group consisting of amino acids, proteins, peptides, nucleotides, anti-obesity drugs, nutraceuticals, dietary supplements, central nervous system stimulants, carotenoids, corticosteroids, elastase inhibitors, anti-fungals, alkylxanthine, oncology therapies, anti-emetics, analgesics, opioids, antipyretics, cardiovascular agents, anti-inflammatory agents, anthelmintics, anti-arrhythmic agents, antibiotics, anticoagulants, antidepressants, antidiabetic agents, antiepileptics, antihistamines, antihypertensive agents, antimuscarinic agents, antimycobacterial agents, antineoplastic agents, immunosuppressants, antithyroid agents, antiviral agents, anxiolytics, sedatives, astringents, alpha-adrenergic receptor blocking agents, beta-adrenoceptor blocking agents, blood products, blood substitutes, cardiac inotropic agents, contrast media, cough suppressants, diagnostic agents, diagnostic imaging agents, diuretics, dopaminergics, haemostatics, immunological agents, lipid regulating agents, muscle relaxants, parasympathomimetics, parathyroid calcitonin and biphosphonates, prostaglandins, radio- pharmaceuticals, sex hormones, anti-allergic agents, stimulants, anoretics, sympathomimetics, thyroid agents, vasodilators, vasomodulator, xanthines, Mu receptor antagonists, Kappa receptor antagonists, non-narcotic analgesics, monoamine uptake inhibitors, adenosine regulating agents, cannabinoid derivatives, Substance P antagonists, neurokinin-1 receptor antagonists, and sodium channel blockers, more particularly wherein said nutraceutical is selected from the group consisting of lutein, folic acid, fatty acids, fruit extracts, vegetable extracts, vitamin supplements, mineral supplements, phosphatidylserine, lipoic acid, melatonin, glucosamine/chondroitin, Aloe Vera, Guggul, glutamine, amino acids, green tea, lycopene, whole foods, food additives, herbs, phytonutrients, antioxidants, flavonoid constituents of fruits, evening primrose oil, flax seeds, fish oils, marine animal oils, and probiotics.

13. The composition of claim 12, wherein:
(a) at least one non-nystatin active agent has an effective average particle size of less than about 2 microns; and/or
(b) at least one non-nystatin active agent has an effective average particle size of greater than about 2 microns.

14. The composition of any one of claims 1 to 13, wherein upon administration the composition redisperses such that the nystatin particles have an effective average particle size selected from the group consisting of less than about 2 microns, less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, and less than about 50 nm; and/or wherein the composition redisperses in a biorelevant media such that the nystatin particles have an effective average particle size selected from the group consisting of less than about 2 microns, less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, and less than about 50 nm.

15. A liquid oral dosage form comprising a composition according to any one of claims 1 to 14.

16. The composition of any one of claims 1 to 14, wherein the composition is formulated for administration selected from the group consisting of oral, pulmonary, rectal, opthalmic, colonic, parenteral, intracisternal, intravaginal, intraperitoneal, local, buccal, nasal, and topical administration; and/or wherein the composition further comprises one or more pharmaceutically acceptable excipients, carriers, or a combination thereof.

17. Use of a composition according to any one of claims 1 to 14 for the manufacture of a medicament for the treatment of a fungal infection.

18. A composition according to any one of claims 1 to 14 for use in the treatment of a fungal infection.

19. A method of making a nanoparticulate composition comprising contacting nystatin particles with at least one surface stabilizer for a time and under conditions sufficient to provide a nanoparticulate nystatin composition having an effective average particle size of less than about 2000 nm,
wherein the surface stabilizer is selected from the group consisting of the sodium salt of 1,2-dipalmitoyl-sn-glycero-3-phosphoethanolamine-N-[amino(polyethyleneglycol)2000]; poly(2-methacryloxyethyl trimethylammonium bromide); a tetrafunctional block copolymer derived from sequential addition of propylene oxide and ethylene oxide to ethylenediamine; sodium deoxycholate; poly(lysine, tryptophan) hydrobromide; povidone-iodine; polyvinylpyrrolidone; distearoyl phosphatidyl ethanolamine : polyethyleneglycol : NH₂; and distearoyl phosphatidyl ethanolamine : polyethyleneglycol.

## Patentansprüche

1. Stabile nanopartikuläre Nystatinzusammensetzung, umfassend:
(a) Partikel von Nystatin oder einem Salz davon; und
(b) mit deren Oberfläche assoziiert oder an diese adsorbiert mindestens einen Oberflächenstabilisator, ausgewählt aus der Gruppe bestehend aus dem Natriumsalz von 1,2-Dipalmitoyl-sn-glycero-3-phosphoethanolamin-N-[amino(polyethylenglycol)2000]; Poly(2-methacryloxyethyltrimethylammoniumbromid); einem tetrafunktionellen Blockcopolymer, abgeleitet aus sequentieller Addition von Propylenoxid und Ethylenoxid an Ethylendiamin; Natriumdesoxycholat; Poly(Lysin, Tryptophan) Hydrobromid; Povidon-Jod; Polyvinylpyrrolidon; Distearoylphosphatidylethanolamin : Polyethylenglycol : NH₂; und Distearoylphosphatidylethanolamin : Polyethylenglycol,
wobei die Nystatinpartikel eine wirksame mittlere Partikelgröße von weniger als 2000 nm haben.

2. Zusammensetzung nach Anspruch 1, formuliert in eine flüssige Darreichungsform, wobei die Darreichungsform eine Viskosität von weniger als etwa 2000 mPa · s, gemessen bei 20 °C, bei einer Scherrate von 0,1 (1/s), hat.

3. Zusammensetzung nach Anspruch 2 mit einer Viskosität bei einer Scherrate von 0,1 (1/s), die ausgewählt ist aus der Gruppe bestehend aus von etwa 2000 mPa · s bis etwa 1 mPa · s, von etwa 1900 mPa · s bis etwa 1 mPa · s, von etwa 1800 mPa · s bis etwa 1 mPa · s, von etwa 1700 mPa · s bis etwa 1 mPa · s, von etwa 1600 mPa · s bis etwa 1 mPa · s, von etwa 1500 mPa · s bis etwa 1 mPa · s, von etwa 1400 mPa · s bis etwa 1 mPa · s, von etwa 1300 mPa · s bis etwa 1 mPa · s, von etwa 1200 mPa · s bis etwa 1 mPa · s, von etwa 1100 mPa · s bis etwa 1 mPa · s, von etwa 1000 mPa · s bis etwa 1 mPa · s, von etwa 900 mPa · s bis etwa 1 mPa · s, von etwa 800 mPa · s bis etwa 1 mPa · s, von etwa 700 mPa · s bis etwa 1 mPa · s, von etwa 600 mPa · s bis etwa 1 mPa · s, von etwa 500 mPa · s bis etwa 1 mPa · s, von etwa 400 mPa · s bis etwa 1 mPa · s, von etwa 300 mPa · s bis etwa 1 mPa · s, von etwa 200 mPa · s bis etwa 1 mPa · s, von etwa 175 mPa · s bis etwa 1 mPa · s, von etwa 150 mPa · s bis etwa 1 mPa · s, von etwa 125 mPa · s bis etwa 1 mPa · s, von etwa 100 mPa · s bis etwa 1 mPa · s, von etwa 75 mPa · s bis etwa 1 mPa · s, von etwa 50 mPa · s bis etwa 1 mPa · s, von etwa 25 mPa · s bis etwa 1 mPa · s, von etwa 15 mPa · s bis etwa 1 mPa · s, von etwa 10 mPa · s bis etwa 1 mPa · s und von etwa 5 mPa · s bis etwa 1 mPa · s.

4. Zusammensetzung nach einem der Ansprüche 2 und 3, wobei die Viskosität der Darreichungsform ausgewählt ist aus der Gruppe bestehend aus weniger als etwa 1/200, weniger als etwa 1/100, weniger als etwa 1/50, weniger als etwa 1/25 und weniger als etwa 1/10 der Viskosität einer Nystatinzusammensetzung, wobei das aktive Mittel in solubilisierter Form vorliegt oder als Partikel mit einer wirksamen mittleren Partikelgröße von größer als zwei Mikron vorliegt, bei etwa der gleichen Nystatinkonzentration pro ml, und/oder wobei die Viskosität der Darreichungsform ausgewählt ist aus der Gruppe, bestehend aus weniger als etwa 5 %, weniger als etwa 10 %, weniger als etwa 15 %, weniger als etwa 20 %, weniger als etwa 25 %, weniger als etwa 30 %, weniger als etwa 35 %, weniger als etwa 40 %, weniger als etwa 45 %, weniger als etwa 50 %, weniger als etwa 55 %, weniger als etwa 60 %, weniger als etwa 65 %, weniger als etwa 70 %, weniger als etwa 75 %, weniger als etwa 80 %, weniger als etwa 85 % und weniger als etwa 90 % der Viskosität einer Nystatinzusammensetzung, wobei das aktive Mittel in solubilisierter Form vorliegt oder als Partikel mit einer wirksamen mittleren Partikelgröße von größer als zwei Mikron vorliegt, bei etwa der gleichen Nystatinkonzentration pro ml.

5. Zusammensetzung nach einem beliebigen der Ansprüche 1-4, wobei das Nystatin ausgewählt ist aus der Gruppe bestehend aus einer kristallinen Phase, einer amorphen Phase, einer semikristallinen Phase, einer semiamorphen Phase und Gemischen davon.

6. Zusammensetzung nach einem beliebigen der Ansprüche 1-5, wobei die wirksame mittlere Partikelgröße der nanopartikulären Nystatinpartikel ausgewählt ist aus der Gruppe bestehend aus weniger als etwa 1500 nm, weniger als etwa 1000 nm, weniger als etwa 900 nm, weniger als etwa 800 nm, weniger als etwa 700 nm, weniger als etwa 600 nm, weniger als etwa 500 nm, weniger als etwa 400 nm, weniger als etwa 300 nm, weniger als etwa 250 nm, weniger als etwa 200 nm, weniger als etwa 100 nm, weniger als etwa 75 nm und weniger als etwa 50 nm.

7. Zusammensetzung nach einem beliebigen der Ansprüche 1-6, wobei:
(a) das Nystatin in einer Menge vorhanden ist, die ausgewählt ist aus der Gruppe bestehend aus von etwa 99,5 Gew.-% bis etwa 0,001 Gew.-%, von etwa 95 Gew.-% bis etwa 0,1 Gew.-% und von etwa 90 Gew.-% bis etwa 0,5 Gew.-%, basierend auf dem gemeinsamen Gesamtgewicht von dem Nystatin und mindestens einem Oberflächenstabilisator, nicht einschließend andere Hilfsstoffe; und/oder
(b) mindestens ein Oberflächenstabilisator in einer Menge vorhanden ist, die ausgewählt ist aus der Gruppe bestehend aus von etwa 0,5 Gew.-% bis etwa 99,999 Gew.-%, von etwa 5,0 Gew.-% bis etwa 99,9 Gew.-% und von etwa 10,0 Gew.-% bis etwa 99,5 Gew.-%, basierend auf dem gemeinsamen Trockengesamtgewicht von dem Nystatin und mindestens einem Oberflächenstabilisator, nicht einschließend andere Hilfsstoffe;
oder wobei die Zusammensetzung mindestens zwei Oberflächenstabilisatoren umfasst.

8. Zusammensetzung nach einem beliebigen der Ansprüche 1 bis 7, wobei die Zusammensetzung bioadhäsiv ist.

9. Zusammensetzung nach einem beliebigen der Ansprüche 1 bis 8, wobei die Zusammensetzung in eine flüssige Darreichungsform formuliert ist und die Menge an Nystatin pro ml gleich ist wie oder größer ist als die Menge an Nystatin pro ml einer Nystatinzusammensetzung, wobei das aktive Mittel in solubilisierter Form vorhanden ist oder als Partikel mit einer wirksamen mittleren Partikelgröße von größer als zwei Mikron vorhanden ist.

10. Zusammensetzung nach einem beliebigen der Ansprüche 1 bis 9, wobei die Zusammensetzung weiterhin eine Nystatinzusammensetzung mit einer wirksamen mittleren Partikelgröße von größer als 2 Mikron umfasst.

11. Zusammensetzung nach einem beliebigen der Ansprüche 1 bis 10, wobei die Zusammensetzung zusätzlich mindestens eine zusätzliche nanopartikuläre Nystatinzusammensetzung mit einer wirksamen mittleren Partikelgröße von weniger als etwa 2 Mikron umfasst,
wobei die zusätzliche nanopartikuläre Nystatinzusammensetzung eine wirksame mittlere Partikelgröße hat, die anders ist als die wirksame mittlere Partikelgröße der nanopartikulären Nystatinzusammensetzung nach Anspruch 1 bis 10.

12. Zusammensetzung nach einem beliebigen der Ansprüche 1 bis 11, zusätzlich umfassend mindestens ein aktives Mittel, das nicht Nystatin ist, insbesondere ausgewählt aus der Gruppe bestehend aus Aminosäuren, Proteinen, Peptiden, Nukleotiden, Wirkstoffen gegen Fettsucht, Nutrazeutika (nutraceuticals), Nahrungsergänzungsmitteln, Stimulanzien des Zentralnervensystems, Carotinoiden, Corticosteroiden, Elastaseinhibitoren, Antimykotika, Alkylxanthin, onkologischen Therapien, Antiemetika, Analgetika, Opioiden, Antipyretika, kardiovaskulären Mitteln, Mitteln gegen Entzündung, Anthelminthika, antiarrhythmischen Mitteln, Antibiotika, Antikoagulanzien, Antidepressiva, antidiabetischen Mitteln, Antiepileptika, Antihistaminika, Mitteln gegen Bluthochdruck, antimuskarinergen Mitteln, antimykobakteriellen Mitteln, antineoplastischen Mitteln, Immunsuppressiva, antithyreoidalen Mitteln, antiviralen Mitteln, Anxiolytika, Sedativa, Adstringenzien, alpha-adrenerger Rezeptor-blockierenden Mitteln, beta-adrenerger Rezeptor-blockierenden Mitteln, Blutprodukten, Blutersatzstoffen, inotrop auf das Herz wirkenden Mitteln, Kontrastmitteln, Hustenunterdrückern, diagnostischen Mitteln, diagnostischen Bildgebungsmitteln, Diuretika, Dopaminergika, Haemostatika, immunologischen Mitteln, Lipid-regulierenden Mitteln, Muskelrelaxantien, Parasympathomimetika, Parathyreoidcalcitonin und Biphosphonaten, Prostaglandinen, Radiopharmazeutika, Geschlechtshormonen, antiallergischen Mitteln, Stimulanzien, Anoretika, Sympathomimetika, Thyreoidmitteln, Vasodilatatoren, Vasomodulator, Xanthinen, Mu-Rezeptorantagonisten, Kappa-Rezeptorantagonisten, nicht-narkotischen Analgetika, Monoaminaufnahmeinhibitoren, Adenosin regulierenden Mitteln, Cannabinoidderivativen, Substanz-P-Antagonisten, Neurokinin-1-Rezeptorantagonisten und Natriumkanalblockern, stärker bevorzugt, wobei das Nutrazeutikum (nutraceutical) ausgewählt ist aus der Gruppe bestehend aus Lutein, Folsäure, Fettsäuren, Fruchtextrakten, Pflanzenextrakten, Vitaminergänzungen, Mineralstoffergänzungen, Phosphatidylserin, Liponsäure, Melatonin, Glucosamin/Chondroitin, Aloe Vera, Guggul, Glutamin, Aminosäuren, grünem Tee, Lycopin, Vollwertkost, Lebensmittelzusatzstoffen, Kräutern, pflanzlichen Nährstoffen, Antioxidantien, Flavonoidbestandteilen von Früchten, Nachtkerzenöl, Flachssamen, Fischölen, Ölen aus Meerestieren und Probiotika.

13. Zusammensetzung nach Anspruch 12, wobei:
(a) mindestens ein aktives Mittel, das nicht Nystatin ist, eine wirksame mittlere Partikelgröße von weniger als etwa 2 Mikron hat; und/oder
(b) mindestens ein aktives Mittel, das nicht Nystatin ist, eine wirksame mittlere Partikelgröße von größer als etwa 2 Mikron hat.

14. Zusammensetzung nach einem beliebigen der Ansprüche 1 bis 13, wobei die Zusammensetzung bei Verabreichung wieder dispergiert, so dass die Nystatinpartikel eine wirksame mittlere Partikelgröße haben, die ausgewählt ist aus der Gruppe bestehend aus weniger als etwa 2 Mikron, weniger als etwa 1900 nm, weniger als etwa 1800 nm, weniger als etwa 1700 nm, weniger als etwa 1600 nm, weniger als etwa 1500 nm, weniger als etwa 1400 nm, weniger als etwa 1300 nm, weniger als etwa 1200 nm, weniger als etwa 1100 nm, weniger als etwa 1000 nm, weniger als etwa 900 nm, weniger als etwa 800 nm, weniger als etwa 700 nm, weniger als etwa 600 nm, weniger als etwa 500 nm, weniger als etwa 400 nm, weniger als etwa 300 nm, weniger als etwa 250 nm, weniger als etwa 200 nm, weniger als etwa 150 nm, weniger als etwa 100 nm, weniger als etwa 75 nm und weniger als etwa 50 nm; und/oder wobei die Zusammensetzung in einem biorelevanten Medium wieder dispergiert, so dass die Nystatinpartikel eine wirksame mittlere Partikelgröße haben, die ausgewählt ist aus der Gruppe bestehend aus weniger als etwa 2 Mikron, weniger als etwa 1900 nm, weniger als etwa 1800 nm, weniger als etwa 1700 nm, weniger als etwa 1600 nm, weniger als etwa 1500 nm, weniger als etwa 1400 nm, weniger als etwa 1300 nm, weniger als etwa 1200 nm, weniger als etwa 1100 nm, weniger als etwa 1000 nm, weniger als etwa 900 nm, weniger als etwa 800 nm, weniger als etwa 700 nm, weniger als etwa 600 nm, weniger als etwa 500 nm, weniger als etwa 400 nm, weniger als etwa 300 nm, weniger als etwa 250 nm, weniger als etwa 200 nm, weniger als etwa 150 nm, weniger als etwa 100 nm, weniger als etwa 75 nm und weniger als etwa 50 nm.

15. Flüssige orale Darreichungsform, umfassend eine Zusammensetzung nach einem beliebigen der Ansprüche 1 bis 14.

16. Zusammensetzung nach einem beliebigen der Ansprüche 1 bis 14, wobei die Zusammensetzung formuliert ist für Verabreichung, ausgewählt aus der Gruppe bestehend aus oraler, pulmonaler, rektaler, ophthalmischer, Darm-, parenteraler, intracistemaler, intravaginaler, intraperitonealer, lokaler, bukkaler, nasaler und topischer Verabreichung; und/oder wobei die Zusammensetzung weiterhin ein oder mehrere pharmazeutisch verträgliche Hilfsstoffe, Träger oder eine Kombination davon umfasst.

17. Verwendung einer Zusammensetzung nach einem beliebigen der Ansprüche 1 bis 14 zur Herstellung eines Medikaments zur Behandlung einer Pilzinfektion.

18. Zusammensetzung nach einem beliebigen der Ansprüche 1 bis 14 zur Verwendung bei der Behandlung einer Pilzinfektion.

19. Verfahren zur Herstellung einer nanopartikulären Zusammensetzung, umfassend das In-Kontakt-Bringen von Nystatinpartikeln mit mindestens einem Oberflächenstabilisator für eine Zeit und unter Bedingungen, die ausreichen, um eine nanopartikuläre Nystatinzusammensetzung mit einer wirksamen mittleren Partikelgröße von weniger als etwa 2000 nm bereitzustellen,
wobei der Oberflächenstabilisator ausgewählt ist aus der Gruppe bestehend aus dem Natriumsalz von 1,2-Dipalmitoyl-sn-glycero-3-phosphoethanolamin-N-[amino(polyethylenglycol)2000]; Poly(2-methacryloxyethyltrimethylammoniumbromid); einem tetrafunktionellen Blockcopolymer, abgeleitet von der sequentiellen Addition von Propylenoxid und Ethylenoxid an Ethylendiamin; Natriumdesoxycholat; Poly(Lysin, Tryptophan) Hydrobromid; Povidon-Jod; Polyvinylpyrrolidon; Distearoylphosphatidylethanolamin : Polyethylenglycol : NH₂; und Distearoylphosphatidylethanolamin : Polyethylenglycol.

## Revendications

1. Composition nanoparticulaire stable de nystatine comprenant :
(a) des particules de nystatine ou d'un sel de celle-ci ; et
(b) associé avec ou adsorbé à la surface de celles-ci, au moins un stabilisant de surface choisi dans le groupe constitué par le sel de sodium de 1,2-dipalmitoyl-sn-glycéro-3-phosphoéthanolamine-N-[amino(polyéthylèneglycol)2000] ; le poly(bromure de 2-méthacryloxyéthyltriméthylammonium) ; un copolymère séquencé tétrafonctionnel dérivé de l'addition séquentielle d'oxyde de propylène et d'oxyde d'éthylène à de l'éthylènediamine ; le désoxycholate de sodium ; le bromhydrate de poly(lysine,tryptophane) ; la povidone iodée ; la polyvinylpyrrolidone ; le distéaroylphosphatidyléthanolamine:polyéthylèneglycol:NH₂ ; et le distéaroylphosphatidyléthanolamine:polyéthylèneglycol,
dans laquelle les particules de nystatine ont une taille moyenne effective de particules de moins de 2000 nm.

2. Composition selon la revendication 1 formulée sous une forme posologique liquide, dans laquelle la forme posologique a une viscosité de moins d'environ 2000 mPa·s, mesurée à 20°C, à une vitesse de cisaillement de 0,1 (l/s).

3. Composition selon la revendication 2, ayant une viscosité à une vitesse de cisaillement de 0,1 (1/s) choisie dans le groupe constitué par d'environ 2000 mPa.s à environ 1 mPa.s, d'environ 1900 mPa.s à environ 1 mPa.s, d'environ 1800 mPa.s à environ 1 mPa.s, d'environ 1700 mPa.s à environ 1 mPa.s, d'environ 1600 mPa.s à environ 1 mPa.s, d'environ 1500 mPa.s à environ 1 mPa.s, d'environ 1400 mPa.s à environ 1 mPa.s, d'environ 1300 mPa.s à environ 1 mPa.s, d'environ 1200 mPa.s à environ 1 mPa.s, d'environ 1100 mPa.s à environ 1 mPa.s, d'environ 1000 mPa.s à environ 1 mPa.s, d'environ 900 mPa.s à environ 1 mPa.s, d'environ 800 mPa.s à environ 1 mPa.s, d'environ 700 mPa.s à environ 1 mPa.s, d'environ 600 mPa.s à environ 1 mPa.s, d'environ 500 mPa.s à environ 1 mPa.s, d'environ 400 mPa.s à environ 1 mPa.s, d'environ 300 mPa.s à environ 1 mPa.s, d'environ 200 mPa.s à environ 1 mPa.s, d'environ 175 mPa.s à environ 1 mPa.s, d'environ 150 mPa.s à environ 1 mPa.s, d'environ 125 mPa.s à environ 1 mPa.s, d'environ 100 mPa.s à environ 1 mPa.s, d'environ 75 mPa.s à environ 1 mPa.s, d'environ 50 mPa.s à environ 1 mPa.s, d'environ 25 mPa.s à environ 1 mPa.s, d'environ 15 mPa.s à environ 1 mPa.s, d'environ 10 mPa.s à environ 1 mPa.s, et d'environ 5 mPa.s à environ 1 mPa.s.

4. Composition selon l'une ou l'autre des revendications 2 et 3, dans laquelle la viscosité de la forme posologique est choisie dans le groupe constitué par moins d'environ 1/200, moins d'environ 1/100, moins d'environ 1/50, moins d'environ 1/25, et moins d'environ 1/10, de la viscosité d'une composition de nystatine dans laquelle l'agent actif est présent sous une forme solubilisée ou présent sous forme de particules ayant une taille moyenne effective de particules de plus de deux microns, à peu près à la même concentration par ml de nystatine et/ou dans laquelle la viscosité de la forme posologique est choisie dans le groupe constitué par moins d'environ 5 %, moins d'environ 10 %, moins d'environ 15 %, moins d'environ 20 %, moins d'environ 25 %, moins d'environ 30 %, moins d'environ 35 %, moins d'environ 40 %, moins d'environ 45 %, moins d'environ 50 %, moins d'environ 55 %, moins d'environ 60 %, moins d'environ 65 %, moins d'environ 70 %, moins d'environ 75 %, moins d'environ 80 %, moins d'environ 85 %, et moins d'environ 90 %, de la viscosité d'une composition de nystatine dans laquelle l'agent actif est présent sous une forme solubilisée ou présent sous forme de particules ayant une taille moyenne effective de particules de plus de deux microns à peu près à la même concentration par ml de nystatine.

5. Composition selon l'une quelconque des revendications 1 à 4, dans laquelle la nystatine est choisie dans le groupe constitué par une phase cristalline, une phase amorphe, une phase semi-cristalline, une phase semi-amorphe, et les mélanges de celles-ci.

6. Composition selon l'une quelconque des revendications 1 à 5, dans laquelle la taille moyenne effective des particules nanoparticulaires de nystatine est choisie dans le groupe constitué par moins d'environ 1500 nm, moins d'environ 1000 nm, moins d'environ 900 nm, moins d'environ 800 nm, moins d'environ 700 nm, moins d'environ 600 nm, moins d'environ 500 nm, moins d'environ 400 nm, moins d'environ 300 nm, moins d'environ 250 nm, moins d'environ 200 nm, moins d'environ 100 nm, moins d'environ 75 nm, et moins d'environ 50 nm.

7. Composition selon l'une quelconque des revendications 1 à 6, dans laquelle :
(a) la nystatine est présente dans une quantité choisie dans le groupe constitué par d'environ 99,5 % à environ 0,001 %, d'environ 95 % à environ 0,1 %, et d'environ 90 % à environ 0,5 %, en poids, rapporté au poids total combiné de la nystatine et d'au moins un stabilisant de surface, sans compter d'autres excipients ; et/ou
(b) au moins un stabilisant de surface est présent dans une quantité choisie dans le groupe constitué par d'environ 0,5 % à environ 99,999 % en poids, d'environ 5,0 % à environ 99,9 % en poids, et d'environ 10,0 % à environ 99,5 % en poids, rapporté au poids sec total combiné de la nystatine et d'au moins un stabilisant de surface, sans compter d'autres excipients,
ou dans laquelle la composition comprend au moins deux stabilisants de surface.

8. Composition selon l'une quelconque des revendications 1 à 7, la composition étant bioadhésive.

9. Composition selon l'une quelconque des revendications 1 à 8, la composition étant formulée sous une forme posologique liquide, et dans laquelle la quantité de nystatine par ml est égale ou supérieure à la quantité de nystatine par ml d'une composition de nystatine dans laquelle l'agent actif est présent sous une forme solubilisée ou présent sous forme de particules ayant une taille moyenne effective de particules de plus de deux microns.

10. Composition selon l'une quelconque des revendications 1 à 9, la composition comprenant en outre une composition de nystatine ayant une taille moyenne effective de particules de plus d'environ deux microns.

11. Composition selon l'une quelconque des revendications 1 à 10, la composition comprenant de plus au moins une composition nanoparticulaire de nystatine supplémentaire ayant une taille moyenne effective de particules de moins d'environ deux microns,
dans laquelle ladite composition nanoparticulaire de nystatine supplémentaire a une taille moyenne effective de particules qui est différente de la taille moyenne effective de particules de la composition nanoparticulaire de nystatine des revendications 1 à 10.

12. Composition selon l'une quelconque des revendications 1 à 11, comprenant de plus au moins un autre agent actif que la nystatine, particulièrement choisi dans le groupe constitué par les acides aminés, les protéines, les peptides, les nucléotides, les médicaments anti-obésité, les nutraceutiques, les compléments alimentaires, les stimulants du système nerveux central, les caroténoïdes, les corticostéroïdes, les inhibiteurs de l'élastase, les antifongiques, les alkylxanthines, les thérapies oncologiques, les antiémétiques, les analgésiques, les opioïdes, les antipyrétiques, les agents cardiovasculaires, les agents anti-inflammatoires, les anthelmintiques, les agents anti-arythmiques, les antibiotiques, les anticoagulants, les antidépresseurs, les agents antidiabétiques, les antiépileptiques, les antihistaminiques, les agents antihypertenseurs, les agents antimuscariniques, les agents antimycobactériens, les agents antinéoplasiques, les immunosuppresseurs, les agents antithyroïdiens, les agents antiviraux, les anxiolytiques, les sédatifs, les astringents, les agents bloquant les récepteurs alpha-adrénergiques, les agents bloquant les récepteurs bêta-adrénergiques, les produits sanguins, les substituts sanguins, les agents inotropes cardiaques, les milieux de contraste, les antitussifs, les agents de diagnostic, les agents d'imagerie de diagnostic, les diurétiques, les dopaminergiques, les hémostatiques, les agents immunologiques, les agents de régulation des lipides, les relaxants musculaires, les parasympathomimétiques, la calcitonine et les biphosphonates parathyroïdiens, les prostaglandines, les produits radiopharmaceutiques, les hormones sexuelles, les agents antiallergiques, les stimulants, les anorexigènes, les sympathomimétiques, les agents thyroïdiens, les vasodilatateurs, les vasomodulateurs, les xanthines, les antagonistes du récepteur Mu, les antagonistes du récepteur Kappa, les analgésiques non-narcotiques, les inhibiteurs de l'assimilation de monoamines, les agents régulant l'adénosine, les dérivés de cannabinoïdes, les antagonistes de la substance P, les antagonistes des récepteurs de la neurokinine-1, et les inhibiteurs sodiques, plus particulièrement dans laquelle ledit nutraceutique est choisi dans le groupe constitué par la lutéine, l'acide folique, les acides gras, les extraits de fruits, les extraits de légumes, les compléments vitaminés, les compléments en minéraux, la phosphatidylsérine, l'acide lipoïque, la mélatonine, la glucosamine/chondroïtine, l'aloe vera, le guggul, la glutamine, les acides aminés, le thé vert, le lycopène, les aliments complets, les additifs alimentaires, les herbes, les phytonutriments, les antioxydants, les constituants flavonoïdes des fruits, l'huile d'onagre, les graines de lin, les huiles de poissons, les huiles d'animaux marins, et les probiotiques.

13. Composition selon la revendication 12, dans laquelle :
(a) au moins un autre agent actif que la nystatine a une taille moyenne effective de particules de moins d'environ deux microns ; et/ou
(b) au moins un autre agent actif que la nystatine a une taille moyenne effective de particules de plus d'environ deux microns.

14. Composition selon l'une quelconque des revendications 1 à 13 dans laquelle, lors d'une administration, la composition se redisperse de telle sorte que les particules de nystatine ont une taille moyenne effective de particules choisie dans le groupe constitué par moins d'environ deux microns, moins d'environ 1900 nm, moins d'environ 1800 nm, moins d'environ 1700 nm, moins d'environ 1600 nm, moins d'environ 1500 nm, moins d'environ 1400 nm, moins d'environ 1300 nm, moins d'environ 1200 nm, moins d'environ 1100 nm, moins d'environ 1000 nm, moins d'environ 900 nm, moins d'environ 800 nm, moins d'environ 700 nm, moins d'environ 600 nm, moins d'environ 500 nm, moins d'environ 400 nm, moins d'environ 300 nm, moins d'environ 250 nm, moins d'environ 200 nm, moins d'environ 150 nm, moins d'environ 100 nm, moins d'environ 75 nm, et moins d'environ 50 nm ; et/ou dans laquelle la composition se redisperse dans un milieu d'importance biologique de telle sorte que les particules de nystatine ont une taille moyenne effective de particules choisie dans le groupe constitué par moins d'environ deux microns, moins d'environ 1900 nm, moins d'environ 1800 nm, moins d'environ 1700 nm, moins d'environ 1600 nm, moins d'environ 1500 nm, moins d'environ 1400 nm, moins d'environ 1300 nm, moins d'environ 1200 nm, moins d'environ 1100 nm, moins d'environ 1000 nm, moins d'environ 900 nm, moins d'environ 800 nm, moins d'environ 700 nm, moins d'environ 600 nm, moins d'environ 500 nm, moins d'environ 400 nm, moins d'environ 300 nm, moins d'environ 250 nm, moins d'environ 200 nm, moins d'environ 150 nm, moins d'environ 100 nm, moins d'environ 75 nm, et moins d'environ 50 nm.

15. Forme posologique orale liquide comprenant une composition selon l'une quelconque des revendications 1 à 14.

16. Composition selon l'une quelconque des revendications 1 à 14, la composition étant formulée pour une administration choisie dans le groupe constitué par l'administration orale, pulmonaire, rectale, ophtalmique, colonique, parentérale, intracisternale, intravaginale, intrapéritonéale, locale, buccale, nasale, et topique ; et/ou la composition comprenant en outre un ou plusieurs excipients ou véhicules pharmaceutiquement acceptables, ou une combinaison de ceux-ci.

17. Utilisation d'une composition selon l'une quelconque des revendications 1 à 14 pour la préparation d'un médicament pour le traitement d'une infection fongique.

18. Composition selon l'une quelconque des revendications 1 à 14 pour utilisation dans le traitement d'une infection fongique.

19. Procédé de préparation d'une composition nanoparticulaire comprenant la mise en contact de particules de nystatine avec au moins un stabilisant de surface pendant une durée et dans des conditions suffisantes pour obtenir une composition nanoparticulaire de nystatine ayant une taille moyenne effective de particules de moins d'environ 2000 nm, dans lequel le stabilisant de surface est choisi dans le groupe constitué par le sel de sodium de 1,2-dipalmitoyl-sn-glycéro-3-phosphoéthanolamine-N-[amino(polyéthylèneglycol)2000] ; le poly(bromure de 2-méthacryloxyéthyltriméthylammonium) ; un copolymère séquencé tétrafonctionnel dérivé de l'addition séquentielle d'oxyde de propylène et d'oxyde d'éthylène à de l'éthylènediamine ; le désoxycholate de sodium ; le bromhydrate de poly(lysine,tryptophane) ; la povidone iodée ; la polyvinylpyrrolidone ; le distéaroylphosphatidyléthanolamine:polyéthylèneglycol:NH₂ ; et le distéaroylphosphatidyléthanolamine:polyéthylèneglycol.
